# EUROPEAN PATENT APPLICATION

(11) **EP 4 223 887 A1**
(43) Date of publication of application: **09.08.2023**
(21) Application number: 21816304.6
(22) Date of filing: 01.10.2021
(51) Int. Cl.: C12Q 1/6883, G01N 33/50, G01N 33/569, G01N 33/68

(54) **METHOD OF PREDICTING PROGRESSIVE COVID-19 SEVERITY BY USING PROTEIN MARKERS IN BLOOD EXOSOMES**

(30) Priority: 02.10.2020 JP 2020168107
(71) Applicant: International Space Medical Co., Ltd., Tokyo, 102-0082 (JP)
(72) Inventor: MIYATO, Mitsuru, Fujisawa-shi, Kanagawa 251-0052 (JP); OCHIYA, Takahiro, Chuo-ku, Tokyo 104-0053 (JP); FUJITA, Yu, Chiba-shi, Chiba 266-0031 (JP); MATSUZAKI, Juntaro, Shinjyuku-ku, Tokyo 160-0018 (JP)
(74) Representative: Griffin, Philippa Jane
(86) International application number: PCT/JP2021/036525
(87) International publication number: WO 2022/071601

(57) **Abstract**

The present invention provides a method for predicting the development of a severe symptom in a COVID-19 patient using a level of RNA of COPB2, KRAS, PRKCB, RHOC, CD147, CAPN2, ECM1, FGG, MFAP4, ADI1, AK1, MGAT1, CLDN3, CRP, UQCRC2, FGA, FGB, FGL1, GPX1, GSK3B, LBP, PDGFC, RAB13, RAP1B, SLC6A4, UBA7, or the like contained in the blood of a patient.

## Description

### Technical Field

The present invention relates to, for example, a method for predicting a risk of development of severe symptom of SARS-CoV-2 infectious disease by detecting exosomal protein in blood.

### Background Art

Severe acute respiratory syndrome coronavirus 2 (SARS-CoV-2) infectious disease (COVID-19) has disseminated worldwide and become pandemic. SARS-CoV-2 is a virus of the genus *Coronavirus* and is a very small particle (diameter: 0.1 nm) composed of a nucleic acid (RNA) that functions as genetic code, a protein capsid that surrounds the nucleic acid, and a lipid bilayer envelope having a spike. Infection with this virus is spreading from person to person by droplet infection in which the virus released from the droplets, such as cough, sneeze, or nasal discharge, of infected individuals enters the mouth or the eyes, or by contact infection in which the hand that has touched the virus in the droplets of infected individuals touches the mouth or the nose.

This infectious disease causes initial symptoms, such as fever or couth, similar to common cold or the like through a long incubation period of 1 to 14 days. The majority of infected individuals are mild cases or asymptomatic inapparent cases. Remaining some COVID-19 patients are of concern and develop serious respiratory problems such as acute respiratory distress syndrome (ARDS), which are further accompanied by myocarditis, angiitis, encephalomeningitis, or the like, resulting in mortal danger.

Currently, the presence or absence of SARS-CoV-2 infection is being actively tested in each country, and PCR tests as well as antigen tests and antibody tests are being conducted. However, these testing methods determine the presence itself of the virus or past infection with the virus and, unfortunately, cannot predict a development of severe symptom in virally infected patient. Although some patients having a pre-existing condition of an underlying disease, for example, cardiovascular disease, respiratory disease, or diabetes mellitus, and pregnant women, aged people, etc., are apt to develop severe symptom, cases with development of severe symptom are also found in healthy young individuals. Thus, there is a demand for a more accurate method for predicting the development of severe symptom.

### Summary of Invention

### Technical Problem

Accordingly, an object of the present invention is to provide a method for predicting a development of severe symptom in SARS-CoV-2-infected patient.

### Solution to Problem

The present inventors collected blood sample from patient who had SARS-CoV-2 positivity determined by PCR tests and manifested moderate symptoms, and then observed the degrees of progression of pathological conditions. Then, as a result of retrospectively analyzing a relationship between the degrees of progression of the pathological conditions of patient and proteins contained in exosomes in the blood samples, it has been found that the levels of exosomal COPB2, KRAS, PRKCB, RHOC, CD147, CAPN2, ECM1, FGG, MFAP4, ADI1, AK1, MGAT1, CLDN3, CRP, UQCRC2, FGA, FGB, FGL1, GPX1, GSK3B, LBP, PDGFC, RAB13, RAP1B, SLC6A4, UBA7, ORM1, RNPEP, ANGPT1, APOB, B4GALT1, BHMT, CPN1, GNAZ, ICAM2, SELL, MAN1A1, SERPINA5, PACSIN2, NCF1B, TMEM59, YWHAB, ABAT, ADH1B, ASL, ASS1, CDH2, CAB39, CPS1, CD226, COL6A3, CUL4A, DSC1, ENTPD5, EIF4A1, FN1, PGC, RHEB, GNAI2, GNB1, GNA13, ITGA2B, ITGB1, ILK, F11R, LTA4H, LIMS1, NAV2, FAM129B, NNMT, NID1, PPIA, PLA1A, PPBP, PECAM1, GP1BB, PCSK9, MENT, SERPINA10, F2RL3, LOX, SFTPB, RAB5B, RALB, REEP6, RETN, AGXT, CCT2, THBD, ISG15, and ZYX derived from patients' blood serve as markers suggesting that COVID-19 patient develops severe symptom or does not develop severe symptom. Accordingly, the present invention provides a method for predicting the development of severe symptom in COVID-19 patient using the above-mentioned proteins contained in exosomes in blood.

As used herein, the "COVID-19 patient" means a subject found to have SARS-CoV-2 infection irrespective of the presence or absence of its symptom or a subject suspected of having SARS-CoV-2 infection. The COVID-19 patient may be, for example, a patient having SARS-CoV-2 positivity determined by a PCR test. SARS-CoV-2 is a coronavirus called severe acute respiratory syndrome coronavirus 2 or 2019 novel coronavirus (2019-nCoV) and is a positive-sense single-stranded RNA virus of 29.9 kb in full length. The gene sequence of the originally developed Wuhan strain (Wuhan-Hu-1) is published under GenBank_ID MN908947. According to the report of Nextstrain, a mutation rate of 25.9 nucleotide mutations/genome/year has been reported, and at least nine nucleotide mutations are considered to occur randomly. Accordingly, as used herein, the term "SARS-CoV-2" includes the Wuhan strain of SARS-CoV-2 and all strains derived therefrom by mutation(s).

In the present specification, a marker protein is selected from COPB2 (COPI coat complex subunit beta 2) (e.g., SEQ ID NO: 11), KRAS (KRAS proto-oncogene) (e.g., SEQ ID NO: 12), PRKCB (Protein kinase C beta type) (e.g., SEQ ID NO: 13), RHOC (Ras homolog family member C) (e.g., SEQ ID NO: 14), CD147 (Basigin, extracellular matrix metalloproteinase inducer (EMMPRIN)) (e.g., SEQ ID NO: 15), CAPN2 (Calpain-2) (e.g., SEQ ID NO: 16), ECM1 (Extracellular matrix protein 1) (e.g., SEQ ID NO: 17), FGG (Fibrinogen gamma chain) (e.g., SEQ ID NO: 18), MFAP4 (microfibril-associated protein 4) (e.g., SEQ ID NO: 19), ADI1 (1,2-dihydroxy-3-keto-5-methylthiopentene dioxygenase, APL1, ARD, Fe-ARD, HMFT1638, MTCBP1, Ni-ARD, SIPL, mtnD), AK1 (Adenylate kinase isoenzyme 1), MGAT1 (Alpha-1,3-mannosyl-glycoprotein 2-beta-N-acetylglucosaminyltransferase), CLDN3 (Claudin 3), CRP (C-reactive protein), UQCRC2 (Cytochrome b-c1 complex subunit 2, mitochondrial), FGA (Fibrinogen alpha chain), FGB (Fibrinogen beta chain), FGL1 (Fibrinogen-like protein 1), GPX1 (Glutathione peroxidase 1), GSK3B (Glycogen synthase kinase 3 beta), LBP (Lipopolysaccharide binding protein), PDGFC (Platelet Derived Growth Factor C), RAB13 (Ras-related protein Rab-13), RAP1B (Ras-related protein Rap-1b), SLC6A4 (Sodium-dependent serotonin transporter), LIBA7 (Ubiquitin Like Modifier Activating Enzyme 7), ORM1 (Orosomucoid 1, Alpha-1-acid glycoprotein 1), RNPEP (Aminopeptidase B), ANGPT1 (Angiopoietin 1), APOB, B4GALT1, BHMT, CPN1, GNAZ, ICAM2, SELL, MAN1A1, SERPINA5, PACSIN2, NCF1B, TMEM59, YWHAB, ABAT, ADH1B, ASL, ASS1, CDH2, CAB39, CPS1, CD226, COL6A3, CUL4A, DSC1, ENTPD5, EIF4A1, FN1, PGC, RHEB, GNAI2, GNB1, GNA13, ITGA2B, ITGB1, ILK, F11R, LTA4H, LIMS1, NAV2, FAM129B, NNMT, NID1, PPIA, PLA1A, PPBP, PECAM1, GP1BB, PCSK9, MENT, SERPINA10, F2RL3, LOX, SFTPB, RAB5B, RALB, REEP6, RETN, AGXT, CCT2, THBD, ISG15, and ZYX. The marker protein is preferably selected from COPB2, KRAS, PRKCB, RHOC, CD147, CAPN2, ECM1, FGG, MFAP4, ADI1, AK1, MGAT1, CLDN3, CRP, UQCRC2, FGA, FGB, FGL1, GPX1, GSK3B, LBP, PDGFC, RAB13, RAP1B, SLC6A4, and UBA7 and more preferably selected from COPB2, KRAS, PRKCB, RHOC, CD147, CAPN2, ECM1, FGG, and MFAP4.

In the present specification, the marker protein may be an isoform, a precursor protein, a mature protein, or a truncated form of any of the marker proteins mentioned above. Further, some amino acids, for example, 1 to 50, 1 to 30, 1 to 20, 1 to 10, 1 to 8, 1 to 5, 1 to 3, 1 to 2, or 1 amino acid(s), may be substituted or deleted, or amino acid(s) absent in the protein thereof (e.g., 1 to 50, 1 to 30, 1 to 20, 1 to 10, 1 to 8, 1 to 5, 1 to 3, 1 to 2, or 1 amino acid(s)) may be added or inserted thereto. The marker protein also includes a protein having an amino acid sequence having 90% or higher, 91% or higher, 92% or higher, 93% or higher, 94% or higher, 95% or higher, 96% or higher, 97% or higher, 98% or higher or approximately 99% or higher identity to one of the marker proteins mentioned above. The identity can be determined by, for example, BLAST. The marker protein described in the present specification includes mutants or variants thereof unless such interpretation in particular is inconsistent.

As used herein, the "blood sample" includes whole blood, plasma, serum, a fractionated or treated blood product such as a hemolysate of whole blood or blood cells, and a dilution or concentrate thereof, and is preferably serum or a dilution thereof.

As used herein, the term ""development of severe symptom"" means score 5 or greater (hospitalized-severe disease) in the following WHO 2020 scoring for COVID-19 cases.

**[Table 1]**

| **Patient** | **Descriptor** | **Score** |
|---|---|---|
| Uninfected | No clinical or virological evidence of infection | 0 |
| Ambulatory | No limitation of activities | 1 |
| | Limitation of activities | 2 |
| Hospitalized-mild disease | No oxygen therapy | 3 |
| | Oxygen by nasal prongs or mask | 4 |
| Hospitalized-severe disease | Non-invasive ventilation of high-flow oxygen | 5 |
| | Intubation and mechanical ventilation | 6 |
| | Ventilation + additional organ support (pressors, RRT, ECMO) | 7 |

| | | |
|---|---|---|
| RRT: renal replacement therapy, ECMO: extracorporeal membrane oxygenation. | | |

As used herein, the "exosomes" refers to an extracellular vesicle of approximately 20 to 200 nm or 50 to 150 nm in diameter that is released from various cells, and is also referred to as EV. Exosome is known to be capable of having various functions including cell-to-cell communication, antigen presentation, and a transport of proteins and nucleic acids such as mRNA and miRNA. In the present specification, preferably, exosomes have CD9 and CD63 on the surface.

In the present specification, a marker RNA is selected from miR-122-5p, SNORD33, AL732437.2, RNU2-29P, CDKN2B-AS1, AL365184.1, AL365184.1, AL365184.1, AL365184.1, AL365184.1, let-7c-5p, miR-21-5p, miR-140-3p, and C5orf66-AS2 which may have, but not limited to, the nucleic acid sequences as set forth, in order, in SEQ ID NOs: 1 to 10.

In the present specification, a marker RNA also includes an isoform or a variant when the marker RNA has such an isoform or a variant. In the present specification, the marker RNA may have, for example, substitution or deletion of some nucleotides, for example, 1 to 5, 1 to 3, 1 to 2, or 1 nucleotide(s), in any of the sequences known as the marker RNAs mentioned above, or may have the addition or insertion of nucleotide(s) absent in any of the marker RNAs mentioned above (e.g., 1 to 5, 1 to 3, 1 to 2, or 1 nucleotide(s)). In the present specification, the marker RNA also includes RNA having a nucleotide sequence having 90% or higher, 91% or higher, 92% or higher, 93% or higher, 94% or higher, 95% or higher, 96% or higher, 97% or higher, 98% or higher or approximately 99% or higher identity to one of the marker RNAs mentioned above. The identity can be determined by, for example, BLAST. The marker RNA described in the present specification includes mutants or variants thereof unless such interpretation in particular is inconsistent.

### Advantageous Effects of Invention

The method, etc., of the present invention enables a prognosis of COVID-19 patient to be predicted and as such, can be used to determine the necessity of hospitalization or determine the need of a monitoring system. Particularly, patient predicted to develop severe symptom can be subjected to proper treatment or procedures by more frequently confirming their symptoms.

### Brief Description of Drawings

[Figure 1] Figure 1 shows a patient recruiting flowchart for cohorts in Examples.
[Figure 2] Figure 2 shows a workflow of the LC-MS identification of proteome from CD9+/CD63+ EV from serum samples of 31 mild COVID-19 patients and 10 uninfected healthy controls. The 31 mild COVID-19 patients were divided into group 1 (mild case; n = 22) and group 2 (severe case; n = 9) according to a course after sample collection.
[Figure 3] Figure 3 is a PCA map of 723 types of proteins from three test subject groups.
[Figure 4] Figure 4 is graphs showing the correlation of COPB2, KRAS, PRKCM, RHOC, CD147, CAPN2, ECM1, FGG, and MFAP4 among three subject groups. P values correspond to trends based on Pearson's correlation analysis. Error bars represent mean ± SEM. The ordinates depict the amounts of the proteins, and the abscissas depict patient groups (uninfected, group 1 (mild case), and group 2 (severe case)).
[Figure 5] Figure 5 is graphs of ROC analysis for nine EV proteins. Numeric values represent evaluated AUC values (95% CI).
[Figure 6] Figure 6 shows Kaplan-Meier curves of nine EV proteins by a log-rank test. The ordinates depict progression-free rates of patient, and "Time (Days)" on the abscissas depicts the number of days elapsed from the date of enrollment. High and low groups were defined using the optimum cutoff values.
[Figure 7] Figure 7 shows a workflow of the NGS determination of exRNA profiles from serum samples of 31 mild COVID-19 patients and 10 uninfected healthy controls. The 31 mild COVID-19 patients were divided into group 1 (mild case; n = 22) and group 2 (severe case; n = 9) according to a course after sample collection.
[Figure 8] Figure 8 is a PCA map for 43 types of transcripts from three test subject groups.

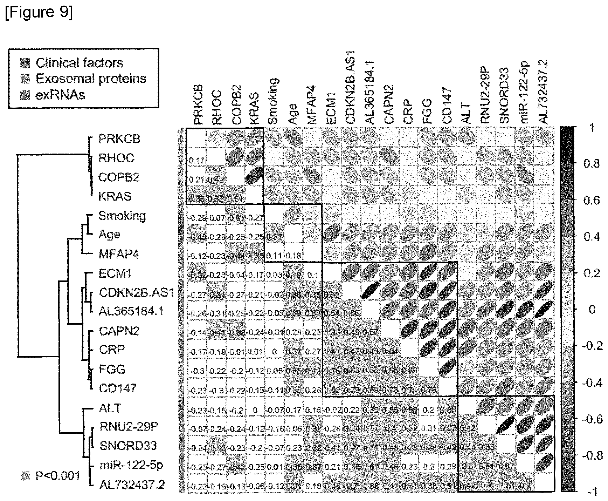

[Figure 9] Colors show Pearson's correlation coefficients. In the upper triangular part, positive correlation is indicated in purple color, and negative correlation is indicated in brown color. The color darkness and ovalization of circles are proportional to the correlation coefficients. The lower triangle depicts actual correlation values, and pink highlights represent P < 0.05. Cluster 1 (PRKCB, RHOC, COPB2, and KRAS) included a group of EV proteins associated with antiviral response. Cluster 2 (smoking, age, and MFPA4) and cluster 3 (CM1, CDKN2B.AS1, AL365184.1, CAPN2, CRP, FGG, and CD147) included a group of coagulation-associated markers. Cluster 4 (ALT, RNU2-29P, SNORD33, miR-122-5p, and AL732437.2) included a group of exRNA associated with hepatic damage.

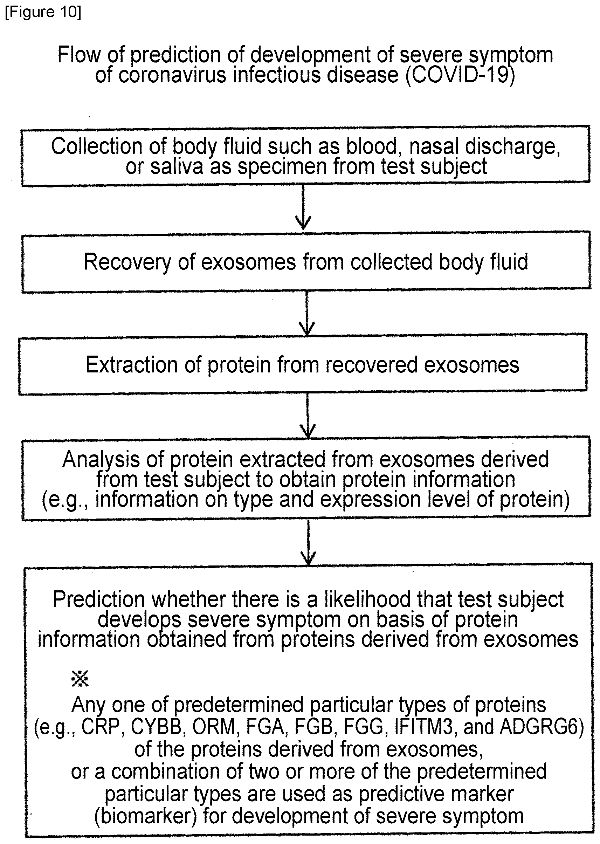

[Figure 10] Figure 10 is a flowchart showing the flow of prediction of development of severe symptom of COVID-19.

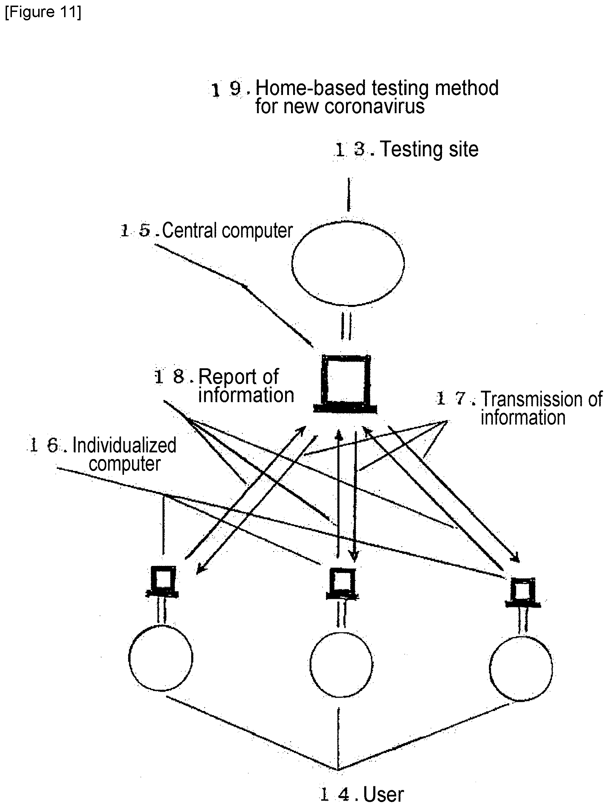

[Figure 11] Figure 11 is a diagram of COVID-19 home care method.

### Description of Embodiments

### 1. Method for determining likelihood of developing severe symptom

In one aspect, the present invention relates to a method for determining a likelihood that a COVID-19 patient develops severe symptom, comprising: measuring a level of one or more types of marker protein present in exosomes in blood derived from the patient; and comparing the measured level of the marker protein with a control RNA level to determine the likelihood that the patient develops severe symptom. The marker protein is selected from the group consisting of the following proteins: COPB2, KRAS, PRKCB, RHOC, CD147, CAPN2, ECM1, FGG, MFAP4, ADI1, AK1, MGAT1, CLDN3, CRP, UQCRC2, FGA, FGB, FGL1, GPX1, GSK3B, LBP, PDGFC, RAB13, RAP1B, SLC6A4, UBA7, ORM1, RNPEP, ANGPT1, APOB, B4GALT1, BHMT, CPN1, GNAZ, ICAM2, SELL, MAN1A1, SERPINA5, PACSIN2, NCF1B, TMEM59, YWHAB, ABAT, ADH1B, ASL, ASS1, CDH2, CAB39, CPS1, CD226, COL6A3, CUL4A, DSC1, ENTPD5, EIF4A1, FN1, PGC, RHEB, GNAI2, GNB1, GNA13, ITGA2B, ITGB1, ILK, F11R, LTA4H, LIMS1, NAV2, FAM129B, NNMT, NID1, PPIA, PLA1A, PPBP, PECAM1, GP1BB, PCSK9, MENT, SERPINA10, F2RL3, LOX, SFTPB, RAB5B, RALB, REEP6, RETN, AGXT, CCT2, THBD, ISG15, and ZYX.

Accordingly, the determination of the likelihood of developing severe symptom according to the present invention may comprise comparing a marker protein level in exosomes derived from the blood of a test subject who is a COVID-19 patient with the marker protein level of a control. For example, in the case where the protein to be measured is COPB2 or KRAS, the patient is determined to maintain a mild condition or to be less likely to develop severe symptom when the level of the protein is higher than that in a healthy person, or the patient can be determined to be likely to develop severe symptom when the level of the protein is not higher than (is lower than or equivalent to) that in a healthy person. In the case where the protein to be measured is PRKCB or RHOC, the patient is determined to be likely to develop severe symptom when the level of the protein is lower than that in a healthy person or at the time of detection of infection in a patient who maintains a mild condition, or the patient is determined to be less likely to develop severe symptom when the level of the protein is not lower than (is higher than or equivalent to) that in a healthy person or at the time of detection of infection in a patient who maintains a mild condition. In the case where the protein to be measured is any of CD147, CAPN2, ECM1, and FGG, the patient is determined to be likely to develop severe symptom when the level of the protein is higher than that in a healthy person or at the time of detection of infection in a patient who maintains a mild condition, or the patient is determined to be less likely to develop severe symptom when the level of the protein is not higher than (is lower than or equivalent to) that in a healthy person or at the time of detection of infection in a patient who maintains a mild condition. In the case where the protein to be measured is MFAP4, the patient is determined to be less likely to develop severe symptom when the level of the protein is lower than that in a healthy person, or the patient is determined to be likely to develop severe symptom when the level of the protein is not lower than (is higher than or equivalent to) that in a healthy person.

The marker protein to be measured in order to determine the likelihood of developing severe symptom can be one or more types selected from COPB2, KRAS, PRKCB, RHOC, CD147, CAPN2, ECM1, FGG, MFAP4, ADI1, AK1, MGAT1, CLDN3, CRP, UQCRC2, FGA, FGB, FGL1, GPX1, GSK3B, LBP, PDGFC, RAB13, RAP1B, SLC6A4, UBA7, ORM1, RNPEP, ANGPT1, APOB, B4GALT1, BHMT, CPN1, GNAZ, ICAM2, SELL, MAN1A1, SERPINA5, PACSIN2, NCF1B, TMEM59, YWHAB, ABAT, ADH1B, ASL, ASS1, CDH2, CAB39, CPS1, CD226, COL6A3, CUL4A, DSC1, ENTPD5, EIF4A1, FN1, PGC, RHEB, GNAI2, GNB1, GNA13, ITGA2B, ITGB1, ILK, F11R, LTA4H, LIMS1, NAV2, FAM129B, NNMT, NID1, PPIA, PLA1A, PPBP, PECAM1, GP1BB, PCSK9, MENT, SERPINA10, F2RL3, LOX, SFTPB, RAB5B, RALB, REEP6, RETN, AGXT, CCT2, THBD, ISG15, and ZYX and can be preferably one or more types selected from COPB2, KRAS, PRKCB, RHOC, CD147, CAPN2, ECM1, FGG, and MFAP4. The RNA to be measured can be, for example, two or more types, three or more types, four or more types, or five or more types.

In the case of using two or more types in combination, preferably, the following combination (a) or (b) can be used:
(a) a combination of two or more types selected from PRKCB, RHOC, COPB2, and KRAS, and
(b) a combination of two or more types selected from ECM1, CAPN2, FGG, and CD147.

As used herein, the "control protein level" means a marker protein level to be compared. In this context, the term "to be compared" means a marker protein level in exosomes in blood from a healthy person or at the time of detection of infection (early after infection) or on admission of a non-severe (mild or asymptomatically maintained) COVID-19 patient. The "control protein level" can be obtained by measuring exosomes sample derived from the blood of a healthy person or exosomes sample derived from the blood of a COVID-19 patient who did not develop severe symptom (mild or asymptomatically maintained) in early times when infection has been detected or on admission, at the same time as measuring the marker protein level in exosomes derived from the blood of a test patient. Alternatively, information on such an already measured negative marker protein level to be compared may be obtained in advance, and this level or a value set in consideration of the level can be used as the control protein level. Such a value may be a cutoff value set by ROC analysis from results of an already conducted test. Alternatively, a sample containing such a preset level of the marker protein may be prepared as a control sample in advance, and the control protein level can be obtained by measurement at the same time as measuring the marker protein level in exosomes derived from the blood of a test patient.

In the case where the method of the present invention comprises a process of measuring a marker protein, the method of the present invention may optionally comprise a step of preparing exosomes from a blood sample derived from a test patient. The preparation of the exosomes can be carried out by use of an arbitrary known method using blood collected from the test subject. Examples of the recovery of the exosomes from a sample such as serum include ultracentrifugation (e.g., Thery C., Curr. Protoc. Cell Biol. (2006) Chapter 3: Unit 3.22.), polymer precipitation, immunoprecipitation, FACS, ultrafiltration, gel filtration, HPLC, and a method of adsorption onto a carrier such as beads using an antibody or lectin. Alternatively, the exosomes may be recovered using a commercially available kit for exosomes isolation.

In the case of adopting a method using an antibody, exosomes can be isolated using a carrier having an anti-CD9 antibody and an anti-CD63 antibody attached thereto, through the use of CD9 and CD63 on the exosomes surface. A step of preparing exosomes may comprise, for example: mixing a blood sample derived from test patient with a carrier having an anti-CD9 antibody and an anti-CD63 antibody attached thereto; and recovering the carrier bound by the exosomes. This step may also comprise, for example, the steps of: washing the carrier bound by the exosomes; and dissociating the exosomes from the carrier.

Among the recovery methods described above, the ultracentrifugation is a standard method that is most commonly used in exosomes isolation. A centrifugal force in the ultracentrifugation can be, for example, 50,000 × g or more, 100,000 × g or more, or 150,000 × g or more and may be 300,000 × g or less, 250,000 × g or less, or 200,000 × g or less. The centrifugation time is not limited and can be, for example, from 30 minutes to 120 minutes, from 60 minutes to 90 minutes, or from 70 minutes to 80 minutes. Before centrifugation, foreign substances may be removed or reduced, if necessary, by filtration through a filter and/or centrifugation with lower centrifugal force.

The recovered exosomes or physical properties of the exosomes can be confirmed according to a known method. For example, it may be visually confirmed under an electron microscope, or particle sizes and the number of particles of the exosomes may be measured by use of a NTA (nano tracking analysis) technique. Alternatively, a presence of the exosomes may be confirmed by confirming expression of a protein and/or a gene capable of serving as a marker for the exosomes.

The protein level may be measured using the prepared exosomes as it is or may be performed after disruption of a membrane with a surfactant such as SDS or RIPA Buffer/RIPA Lysis Buffer. In the case of using SDS, a protein is denatured. In the case of using RIPA Buffer/RIPA Lysis Buffer, an undenatured protein sample can be prepared. Alternatively, a protein may be further extracted from the prepared exosomes and measured. Accordingly, the method of the present invention may optionally comprise extracting and/or purifying a protein from the prepared exosomes. In the case of extracting a protein, a commercially available exosomal protein extraction kit (Cosmo Bio Co., Ltd.), ExoMS Surface Protein Capture Kit (System Biosciences, LLC), or the like can be used for the extraction.

The measurement of a protein level is not particularly limited as long as the method is capable of measuring an amount of the protein. A general method employs a substance that specifically binds to a marker protein. Examples of the "substance that specifically binds to the marker protein" can include antibodies and antigen binding fragments thereof, aptamers, ligands/receptors and binding fragments thereof, and their fusion products with other substances. The "antigen binding fragment" is a protein or a peptide containing a portion of an antibody (partial fragment) and means a protein or a peptide that retains the action (immunoreactivity and binding activity) of the antibody on its antigen. Examples of such an immunoreactive fragment can include F(ab')₂, Fab', Fab, Fab3, single-chain Fv (hereinafter, referred to as "scFv"), (tandem) bispecific single-chain Fv (sc(Fv)₂), single-chain triple body, nanobody, divalent VHH, pentavalent VHH, minibody, (double-chain) diabody, tandem diabody, bispecific tribody, bispecific bibody, dual affinity re-targeting molecule (DART), triabody (or tribody), tetrabody (or [sc(Fv)2]2) or (scFv-SA)₄), disulfide-stabilized Fv (hereinafter, referred to as "dsFv"), compact IgG, heavy chain antibody, and polymers thereof (see Nature Biotechnology, 29 (1): 5-6 (2011); Maneesh Jain et al., TRENDS in Biotechnology, 25 (7) (2007): 307-316; and Christoph Stein et al., Antibodies (1): 88-123 (2012)). In the present specification, the antibody and the immunoreactive fragment may be monospecific, bispecific, trispecific, or multispecific.

Typically, a measurement of a protein level is performed by determining a binding level of a marker protein bound to a substance that specifically binds to the marker protein. The "binding level" to be measured can be an amount of such a substance bound, the number of bound molecules, or a binding ratio, or a numeric value that indicates such a factor (e.g., a measurement value itself such as measured fluorescence intensity). In this case, a labeled substance may be used as the substance that specifically binds to the marker protein, or the marker protein may be labeled for use. In general, a standard sample is measured at the same time, and a value calculated by preparing a standard curve or a calibration curve on the basis of the standard sample, or a numeric value normalized by using the standard sample level as an index is employed as the binding level.

Accordingly, the method of the present invention may comprise, for example:
(a) contacting a substance that binds to at least one marker protein with a protein in exosomes derived from the blood of patient;
(b) determining a binding level of the marker protein in the exosomes bound to the substance that binds to the marker protein; and
(c) determining a marker protein level in the exosomes from the measured binding level.

In the case of using an antibody or an antigen binding fragment thereof as a substance that binds to a marker protein, a measurement of binding can be based on a known detection and/or measurement method. The binding can be measured by, for example, labeled immunoassay such as enzyme immunoassay (EIA), simple EIA, enzyme-linked immunosorbent assay (ELISA), radioimmunoassay (RIA), or fluorescence immunoassay (FIA); immunoblotting such as Western blotting; immunochromatography such as gold colloid aggregation; chromatography such as ion-exchange chromatography or affinity chromatography; turbidimetric immunoassay (TIA); nephelometric immunoassay (NIA); colorimetry; latex immunoturbidimetric assay (LIA); counting immunoassay (CIA); chemiluminescence immunoassay (CLIA and CLEIA); precipitation reaction; surface plasmon resonance (SPR); resonant mirror detection (RMD); or comparative interference.

Specifically, a level of the marker protein can be determined by contacting a test sample (sample) with the antibody of the present invention or the antigen binding fragment thereof immobilized on a solid phase, washing the solid phase, then adding thereto a labeled antibody capable of binding to the marker protein, then washing off unbound antibodies, and detecting the label on the antibody or measuring an amount of the label (e.g., the intensity of the label). In the case of performing immunochromatography, the level of the marker protein can be determined by contacting a sample with a non-immobilized labeled first antibody capable of binding to the marker protein, then contacting the mixture with a carrier on which a second antibody or antigen binding fragment thereof capable of binding to the marker protein is immobilized at a particular site, and detecting the labeled antibody or measuring the amount of the label (e.g., the intensity of the label) at the site.

Examples of a labeling method can include radioisotope (RI) labeling, fluorescent labeling, and enzymatic labeling. Examples of the radioisotope for the RI labeling can include 32P, 131I, 35S, 45Ca, 3H, and 14C. Examples of a fluorescent dye for the fluorescent labeling can include DAPI, SYTOX(R) Green, SYTO(R) 9, TO-PRO(R)-3, Propidium Iodide, Alexa Fluor(R) 350, Alexa Fluor(R) 647, Oregon Green(R), Alexa Fluor(R) 405, Alexa Fluor(R) 680, Fluorescein (FITC), Alexa Fluor(R) 488, Alexa Fluor(R) 750, Cy(R) 3, Alexa Fluor(R) 532, Pacific Blue(TM), Pacific Orange(TM), Alexa Fluor(R) 546, Coumarin, Tetramethylrhodamine (TRITC), Alexa Fluor(R) 555, BODIPY(R) FL, Texas Red(R), Alexa Fluor(R) 568, Pacific Green(TM), Cy(R) 5, and, Alexa Fluor(R) 594. For the enzymatic labeling, biotin (biotin-16-dUTP, biotin-11-dUTP, etc.), digoxigenin (DIG; naturally occurring steroid) (deoxyuridine-5'-triphosphate), alkaline phosphatase, or the like can be used.

The step of determining a marker protein level according to the present invention may employ a composition or a device having a substance that binds to the marker protein as described below.

As used herein, the phrase "specifically bind" to a protein means that a substance binds to a nucleic acid having a marker protein sequence with substantially higher affinity than that for a protein having another amino acid sequence. In this context, the "substantially high affinity" means affinity to the extent that the marker protein can be distinguishably detected from a protein having another amino acid sequence. Another amino acid sequence preferably differs distinguishably from the marker protein sequence and may be an amino acid sequence having 50% or lower, 40% or lower, 30% or lower, 20% or lower, or 10% or lower identity thereto. The substantially high affinity may be, for example, an amount of the substance bound to the marker protein which is 3 or more times, 4 or more times, 5 or more times, 6 or more times, 7 or more times, 8 or more times, 9 or more times, 10 or more times, 15 or more times, 20 or more times, 30 or more times, or 50 or more times an amount of the substance bound to another amino acid sequence.

The likelihood of developing severe symptom according to the present invention may be determined using a level of marker RNA in blood derived from patient, in addition to the marker proteins mentioned above. The method may further comprise measuring a level of one or more types of marker RNA in blood sample derived from patient, wherein the likelihood of developing severe symptom is determined by using the marker protein level and the marker RNA level in combination. Specifically, in the method, the patient is determined to be likely to develop severe symptom when the measured level of the marker RNA is higher than the marker RNA level of a control. The marker RNA is selected from the group consisting of the following RNAs: miR-122-5p, SNORD33, AL732437.2, RNU2-29P, CDKN2B-AS1, AL365184.1, AL365184.1, AL365184.1, AL365184.1, AL365184.1, let-7c-5p, miR-21-5p, miR-140-3p, and C5orf66-AS2.

The method of the present invention may optionally comprise extracting RNA from a blood of COVID-19 patient. The RNA can be extracted using a commercially available RNA extraction kit (e.g., miRNeasy Mini Kit or QlAzol and miRNeasy Mini Kit (all from Qiagen N.V., Hilden, Germany)) according to manufacturer's protocol.

A marker RNA to be measured in order to determine a likelihood of developing severe symptom can be one or more types selected from miR-122-5p, SNORD33, AL732437.2, RNU2-29P, CDKN2B-AS1, AL365184.1, AL365184.1, AL365184.1, AL365184.1, AL365184.1, let-7c-5p, miR-21-5p, miR-140-3p, and C5orf66-AS2 and can be preferably one or more types selected from miR-122-5p, SNORD33, AL732437.2, RNU2-29P, CDKN2B-AS1, and AL365184.1. The RNA to be measured, can be, for example, two or more types, three or more types, four or more types, or five or more types.

In the case of using two or more types in combination, preferably, the following combination (a) or (b) can be used:
(a) a combination of CDKN2B-AS1 and AL365184.1, and
(b) a combination of two or more types selected from miR-122-5p, SNORD33, AL732437.2, and RNU2-29P.

According to the findings of the present inventors, COVID-19 patient is apt to develop severe symptom when such an RNA level in a blood of COVID-19 patient is higher than that in an uninfected healthy person or at the time of detection of infection in COVID-19 patient who maintains a mild condition in a course after the detection of infection. Accordingly, a determination of a likelihood of developing severe symptom according to the present invention can be performed by comparing the marker RNA level in a blood sample of a test subject who is a COVID-19 patient with the RNA level of a control. The test subject is determined to be likely to develop severe symptom when the marker RNA level in the blood sample derived from the test subject is higher than that of a control. The test subject can be determined to be less likely to develop severe symptom or to be likely to maintain a mild condition when the marker RNA level in the sample derived from the test subject is not higher than (is lower than or equivalent to) that of a control.

As used herein, the "marker RNA level of a control" means a negative marker RNA level to be compared. In this context, the "negative level to be compared" means a marker RNA level in a blood sample from a healthy person or at the time of detection of infection (early after infection) or on admission of non-severe (mild or asymptomatically maintained) COVID-19 patient. The "marker RNA level of a control" can be obtained by measuring, as a control, a blood sample of a healthy person or a blood sample of non-severe (mild or asymptomatically maintained) COVID-19 patient in early times when infection has been detected or on admission, at the same time as measuring the marker RNA level in a blood sample of test patient. Alternatively, information on such an already measured negative marker RNA level to be compared may be obtained in advance, and this level or a value set in consideration of the level can be used as the marker RNA level of a control. Such a value may be, for example, a cutoff value set by ROC analysis from results of an already conducted test. Alternatively, a sample containing such a preset level of the marker RNA may be prepared as a control sample in advance, and the marker RNA level of a control can be obtained by measurement at the same time as measuring the marker RNA level in a blood sample of test patient.

A measurement of a RNA level is not particularly limited as long as a method is capable of measuring an amount of the RNA. A general method employs a substance that specifically binds to a marker RNA. As one example, the "substance that specifically binds to the marker RNA" can be a nucleic acid molecule and is preferably a nucleic acid molecule having a sequence complementary to the marker RNA. The nucleic acid molecule having a sequence complementary to the marker RNA can specifically bind to the marker RNA by hybridization. As used herein, the "nucleic acid" may include DNA, RNA, or an artificially developed nucleic acid (including a bridged nucleic acid such as PNA or locked nucleic acid (2',4'-BNA)), or a combination thereof. The nucleic acid molecule that specifically binds to the marker RNA may at least partially contain an artificially designed sequence (e.g., a sequence for labeling or tagging).

The "probe" typically has a sequence complementary to a marker RNA sequence and is a nucleic acid molecule that is used for measuring binding to the marker RNA sequence. The probe is usually a nucleic acid molecule of 10 to 30 mer, 10 to 20 mer, etc., capable of specifically binding to the marker RNA. Examples of a method for measuring a binding level between the marker RNA and the probe can include Southern hybridization, Northern hybridization, dot hybridization, fluorescence *in situ* hybridization (FISH), microarrays, and ASO. Specifically, a method using GeneChip(TM) miRNA Array Strip (Thermo Fisher Scientific K.K.) or Agilent miRNA microarray (Agilent Technologies, Inc.) can be used.

A measurement of an RNA level can be performed by determining a binding level of a marker RNA bound to a substance that specifically binds to the marker RNA. The "binding level" to be measured can be an amount of such a substance bound, the number of bound molecules, or a binding ratio, or a numeric value that indicates such a factor (e.g., a measurement value itself such as measured fluorescence intensity). In this case, a labeled substance may be used as the substance that specifically binds to the marker RNA, or the marker RNA may be labeled for use. In general, a standard sample is measured at the same time, and a value calculated from a measurement value of a measurement sample by preparing a standard curve or a calibration curve on the basis of the standard sample, or a numeric value normalized by using the standard sample level as an index is employed as the binding level.

Examples of a labeling method can include radioisotope (RI) labeling, fluorescent labeling, and enzymatic labeling. Examples of the radioisotope for the RI labeling can include 32P, 131I, 35S, 45Ca, 3H, and 14C. Examples of the fluorescent dye for the fluorescent labeling can include DAPI, SYTOX(R) Green, SYTO(R) 9, TO-PRO(R) -3, Propidium Iodide, Alexa Fluor(R) 350, Alexa Fluor(R) 647, Oregon Green(R), Alexa Fluor(R) 405, Alexa Fluor(R) 680, Fluorescein (FITC), Alexa Fluor(R) 488, Alexa Fluor(R) 750, Cy(R) 3, Alexa Fluor(R) 532, Pacific Blue(TM), Pacific Orange(TM), Alexa Fluor(R) 546, Coumarin, Tetramethylrhodamine (TRITC), Alexa Fluor(R) 555, BODIPY(R) FL, Texas Red(R), Alexa Fluor(R) 568, Pacific Green(TM), Cy(R) 5, and, Alexa Fluor(R) 594. For the enzymatic labeling, biotin (biotin-16-dUTP, biotin-11-dUTP, etc.), digoxigenin (DIG; naturally occurring steroid) (deoxyuridine-5'-triphosphate), alkaline phosphatase, or the like can be used.

The method of the present invention may comprise, for example, the following (a) to (c):
(a) contacting a nucleic acid molecule (probe) that binds to a nucleotide sequence of at least one marker RNA or a portion thereof with a blood sample of a patient;
(b) measuring a binding level of the marker RNA in the blood sample bound to a probe; and
(c) determining a marker RNA level in the blood sample from the measured binding level.

In the method, a composition, a kit, or a device having the nucleic acid molecule (probe) described in the present specification may be used instead of the nucleic acid molecule (probe) that binds to the nucleotide sequence of at least one marker RNA or a portion thereof.

Alternatively, an RNA level can be measured by use of a method using PCR and may be measured, for example, by performing qPCR, ARMS (amplification refractory mutation system), RT-PCR (reverse transcriptase-PCR), or nested PCR using a nucleic acid (primer) that specifically binds to the marker RNA. Alternatively, Invader(R) method may be used. For example, a method using GenoExplorer(TM) miRNA qRT-PCR Kit (GenoSensor Corporation) can be used together with proper primers. The "primer" is usually a nucleic acid molecule of 10 to 30 mer (preferably 17 to 25 mer, 15 to 20 mer, etc.) that is used for nucleic acid amplification, and at least partially has a sequence (preferably of 7 mer or longer, 8 mer or longer, 9 mer or longer, or 10 mer or longer) complementary to a terminal sequence of the marker RNA.

Accordingly, an RNA level may be measured by the following steps:
(a) amplifying the whole or a portion of a marker RNA in a blood sample of patient using the blood sample of the patient as a template and a nucleic acid molecule (primer) capable of specifically binding to the marker RNA;
(b) measuring a level of the amplified nucleic acid molecule; and
(c) determining a marker RNA level in the blood sample from the level of the amplified nucleic acid molecule.

An amplification of the whole or a portion of a marker RNA in a blood sample of a patient can be carried out through PCR reaction or the like with the blood sample as a template. A level of the amplified nucleic acid can be measured by dot blot hybridization, surface plasmon resonance (SPR), PCR-RFLP, *in situ* RT-PCR, PCR-SSO (sequence specific oligonucleotide), PCR-SSP, AMPFLP (amplifiable fragment length polymorphism), MVR-PCR, PCR-SSCP (single strand conformation polymorphism).

As used herein, the phrase "specifically bind" to RNA means that the substance binds to a nucleic acid having a marker RNA sequence with substantially higher affinity than that for a nucleic acid having another nucleotide sequence. In this context, the "substantially high affinity" means affinity to the extent that the nucleic acid having a marker RNA sequence can be distinguishably detected from a nucleic acid having another nucleotide sequence. Another nucleotide sequence preferably differs distinguishably from the marker RNA sequence and may be a nucleotide sequence having 50% or lower, 40% or lower, 30% or lower, 20% or lower, or 10% or lower identity thereto. The substantially high affinity may be, for example, an amount of the substance bound to the marker RNA which is 3 or more times, 4 or more times, 5 or more times, 6 or more times, 7 or more times, 8 or more times, 9 or more times, 10 or more times, 15 or more times, 20 or more times, 30 or more times, or 50 or more times an amount of the substance bound to another nucleotide sequence.

The method of the present invention may be performed by further using age, smoking index, a CRP value in blood, and/or an ALT value in blood, in addition to the marker proteins and the marker RNAs mentioned above. Specifically, a likelihood of developing severe symptom may be determined by using age, smoking index, a CRP value in blood, and/or an ALT value in blood in combination with the marker protein level, or a likelihood of developing severe symptom may be determined by using age, smoking index, a CRP value in blood, and/or an ALT value in blood in combination with the marker protein level and the marker RNA level. In this context, the patient is determined to be likely to develop severe symptom when all of the age, the smoking index, the CRP, and the ALT have a higher numeric value than that of a control which is a healthy person or maintains a mild condition, or the patient is determined to be less likely to develop severe symptom when all of the age, the smoking index, the CRP, and the ALT do not have a higher numeric value (have a lower or equivalent numeric value) than that of a control which is a healthy person or a mild case. The smoking index, CRP, and ALT can be determined or measured by routine methods.

Accordingly, the prediction of the development of severe symptom according to the present invention may comprise determining the development of severe symptom using a combination selected from the following (a) to (d):
(a) two or more factors selected from the group consisting of PRKCB, RHOC, COPB2, and KRAS,
(b) two or more factors selected from the group consisting of smoking index, age, and MFAP4,
(c) two or more factors selected from the group consisting of CDKN2B-AS1, AL365184.1, ECM1, CAPN2, CRP, FGG, and CD147,
(d) two or more types of factors selected from the group consisting of ALT, RNU2-29P, SNORD33, miR-122-5p, and AL732437.2.

Whether a marker level in a sample derived from a test subject is higher or lower than a control level to be compared can be determined by statistical analysis. Statistical significance can be determined by a statistical approach such as T-test, F-test, or chi-square test and can be determined, for example, by comparing two or more samples to determine a confidence interval and/or a p value (Dowdy and Wearden, Statistics for Research, John Wiley & Sons, New York, 1983). The confidence interval of the present invention may be, for example, 90%, 95%, 98%, 99%, 99.5%, 99.9% or 99.99%. The p value may be, for example, 0.1, 0.05, 0.025, 0.02, 0.01, 0.005, 0.001, 0.0005, 0.0002 or 0.0001.

Throughout the present specification, the "level" means an index related to an abundance converted into a numeric value and includes, for example, a concentration, an amount, or an index (preferably a numeric index) that can be used instead thereof. Accordingly, the level may be a measurement value itself such as fluorescence intensity or may be a value converted into a concentration. The level may be an absolute numeric value (abundance, abundance per unit area, etc.) or may be a relative numeric value, if necessary, compared with an established comparative control.

In the present specification, a method for determining a likelihood of developing severe symptom may be used as a method for assessing or evaluating a likelihood of developing severe symptom, a method for predicting the presence or absence of a development of severe symptom, a method for assessing, determining, or evaluating a likelihood of not developing severe symptom, or a method for providing information for performing these methods.

As used herein, the "method for determining a likelihood" comprises the method for monitoring change in the likelihood of developing severe symptom unless such interpretation is inconsistent. Accordingly, as used herein, the phrase "determine a likelihood" may be interpreted as monitoring change in the likelihood of developing severe symptom unless such interpretation in particular is inconsistent. The determination of the likelihood in the monitoring method may be performed continuously or intermittently.

The method for determining the likelihood according to the present invention may be performed *in vivo, ex vivo,* or *in vitro* and is preferably performed *ex vivo* or *in vitro.*

A determination of a likelihood means that course or outcome of condition of a patient is thereby predicted, and does not mean that the course or outcome of condition can be determined with 100% accuracy. The term "be likely to develop severe symptom" means an increased likelihood of causing development of severe symptom and does not mean a higher incidence with reference to the case of not causing development of severe symptom. Specifically, results of determining the likelihood mean that a patient with an elevated level of the marker RNA is more apt to develop severe symptom than a patient who does not exhibit such a feature.

The method for determining a likelihood of developing a severe symptom according to the present invention may comprise subjecting COVID-19 patient determined to be likely to develop severe symptom to prophylactic procedures against the development of severe symptom. Examples of such prophylactic procedures against the development of severe symptom can include administration of vaccines, therapeutic drugs, or prophylactic drugs, treatment or procedures with ventilators, ECMO, or IMPELLA, and elevated frequencies of monitoring of patients' symptoms (e.g., once or more a day, twice or more a day, and three times or more a day).

### 2. Marker for development of severe symptom of COVID-19

In an alternative aspect, the present invention relates to a marker for severe COVID-19 which is at least one type of protein selected from COPB2, KRAS, PRKCB, RHOC, CD147, CAPN2, ECM1, FGG, MFAP4, ADI1, AK1, MGAT1, CLDN3, CRP, UQCRC2, FGA, FGB, FGL1, GPX1, GSK3B, LBP, PDGFC, RAB13, RAP1B, SLC6A4, UBA7, ORM1, RNPEP, ANGPT1, APOB, B4GALT1, BHMT, CPN1, GNAZ, ICAM2, SELL, MAN1A1, SERPINA5, PACSIN2, NCF1B, TMEM59, YWHAB, ABAT, ADH1B, ASL, ASS1, CDH2, CAB39, CPS1, CD226, COL6A3, CUL4A, DSC1, ENTPD5, EIF4A1, FN1, PGC, RHEB, GNAI2, GNB1, GNA13, ITGA2B, ITGB1, ILK, F11R, LTA4H, LIMS1, NAV2, FAM129B, NNMT, NID1, PPIA, PLA1A, PPBP, PECAM1, GP1BB, PCSK9, MENT, SERPINA10, F2RL3, LOX, SFTPB, RAB5B, RALB, REEP6, RETN, AGXT, CCT2, THBD, ISG15, and ZYX. Among them, at least one type of protein selected from COPB2, KRAS, PRKCB, RHOC, CD147, CAPN2, ECM1, FGG, MFAP4, ADI1, AK1, MGAT1, CLDN3, CRP, UQCRC2, FGA, FGB, FGL1, GPX1, GSK3B, LBP, PDGFC, RAB13, RAP1B, SLC6A4, and UBA7 is preferred, and at least one type of protein selected from COPB2, KRAS, PRKCB, RHOC, CD147, CAPN2, ECM1, FGG, and MFAP4 is more preferred.

As used herein, the "marker" means biomolecule whose expression or abundance level in a test subject suggests an already developed particular disease, condition or symptom, or suggests a risk of bringing about a particular disease, condition or symptom in the future. In other words, the marker is an index for determining or predicting a current or future particular disease, condition or symptom, or a molecule that is measured as the index. Specifically, in the case where the marker according to the present invention is any of COPB2, KRAS, PRKCB, RHOC, and MFAP4, a low abundance level of the molecule indicates a high risk of development of severe symptom of COVID-19. In the case where the marker according to the present invention is any of CD147, CAPN2, ECM1, and FGG, a high abundance level of the molecule indicates a high risk of severe COVID-19.

### 3. Composition or kit for prediction of severe COVID-19

In an alternative aspect, the present invention relates to a composition for a prediction of development of severe symptom of COVID-19, comprising a substance capable of binding to at least one type of protein selected from COPB2, KRAS, PRKCB, RHOC, CD147, CAPN2, ECM1, FGG, MFAP4, ADI1, AK1, MGAT1, CLDN3, CRP, UQCRC2, FGA, FGB, FGL1, GPX1, GSK3B, LBP, PDGFC, RAB13, RAP1B, SLC6A4, UBA7, ORM1, RNPEP, ANGPT1, APOB, B4GALT1, BHMT, CPN1, GNAZ, ICAM2, SELL, MAN1A1, SERPINA5, PACSIN2, NCF1B, TMEM59, YWHAB, ABAT, ADH1B, ASL, ASS1, CDH2, CAB39, CPS1, CD226, COL6A3, CUL4A, DSC1, ENTPD5, EIF4A1, FN1, PGC, RHEB, GNAI2, GNB1, GNA13, ITGA2B, ITGB1, ILK, F11R, LTA4H, LIMS1, NAV2, FAM129B, NNMT, NID1, PPIA, PLA1A, PPBP, PECAM1, GP1BB, PCSK9, MENT, SERPINA10, F2RL3, LOX, SFTPB, RAB5B, RALB, REEP6, RETN, AGXT, CCT2, THBD, ISG15, and ZYX. Alternatively, the present invention may provide a kit for the prediction of severe COVID-19, comprising a substance capable of binding to at least one type of protein selected from COPB2, KRAS, PRKCB, RHOC, CD147, CAPN2, ECM1, FGG, MFAP4, ADI1, AK1, MGAT1, CLDN3, CRP, UQCRC2, FGA, FGB, FGL1, GPX1, GSK3B, LBP, PDGFC, RAB13, RAP1B, SLC6A4, UBA7, ORM1, RNPEP, ANGPT1, APOB, B4GALT1, BHMT, CPN1, GNAZ, ICAM2, SELL, MAN1A1, SERPINA5, PACSIN2, NCF1B, TMEM59, YWHAB, ABAT, ADH1B, ASL, ASS1, CDH2, CAB39, CPS1, CD226, COL6A3, CUL4A, DSC1, ENTPD5, EIF4A1, FN1, PGC, RHEB, GNAI2, GNB1, GNA13, ITGA2B, ITGB1, ILK, F11R, LTA4H, LIMS1, NAV2, FAM129B, NNMT, NID1, PPIA, PLA1A, PPBP, PECAM1, GP1BB, PCSK9, MENT, SERPINA10, F2RL3, LOX, SFTPB, RAB5B, RALB, REEP6, RETN, AGXT, CCT2, THBD, ISG15, and ZYX.

Any substance described as the "substance capable of binding to a marker protein" in "1. Method for determining likelihood of developing severe symptom" mentioned above can be used as a substance capable of binding to a marker protein. A kit can be, for example, an immunochemical measurement kit comprising a solid phase or a hapten on which an antibody or a binding fragment thereof capable of binding to the marker protein is immobilized, and a labeled antibody or binding fragment thereof capable of binding to the marker protein. Alternatively, the kit of the present invention can be an immunochemical measurement kit comprising an antibody or a binding fragment thereof capable of binding to the marker protein, and a substance (e.g., an antibody or a binding fragment thereof) capable of binding to the antibody or the binding fragment thereof.

A composition or a kit for a prediction of severe COVID-19 may optionally comprise a buffer solution or the like for stably preserving a substance capable of binding to a marker protein. The composition or the kit for the prediction of severe COVID-19 of the present invention can be based on any known detection and/or measurement method mentioned above.

A composition or a kit for a prediction of severe COVID-19 may be directed to determining a binding level of a marker protein to a substance that specifically binds to the marker protein. In this case, the composition or the kit may be used together with a system for detecting binding to the marker protein by fluorescence or the like.

The composition or the kit may further comprise substances capable of measuring one or more types, two or more types, three or more types, four or more types, or five or more types of marker RNAs described above.

The composition or the kit may comprise, for example, the following combinations:
(a) a combination of two or more types selected from a substance capable of measuring PRKCB, a substance capable of measuring RHOC, a substance capable of measuring COPB2, and a substance capable of measuring KRAS,
(b) a combination of two or more types selected from a substance capable of measuring ECM1, a substance capable of measuring CAPN2, a substance capable of measuring FGG, and a substance capable of measuring CD147.

The kit of the present invention may optionally comprise a coloring reagent, a reagent for termination of reaction, a standard antigen reagent, a reagent for specimen pretreatment, a blocking reagent, etc. The kit of the present invention comprising a labeled substance may comprise a substrate that reacts with the label. The kit of the present invention may comprise a package, such as a paper box or a plastic case, which houses a constituent of the kit, and an instruction manual, etc. A kit may be comprised in one kit comprising different components packed in separate containers, or may be a kit comprising only the substance capable of binding to the marker protein while other components are provided aside from the kit.

### 4. Device for prediction of severe COVID-19

In the present specification, a device may comprise an array, beads, a chip, an immunochromatographic plate or a column, etc., having substances bound thereto capable of binding to one or more types, two or more types, three or more types, four or more types, or five types of marker proteins. This device may be the one for measuring binding levels between the marker proteins mentioned above and substances that specifically bind to the marker proteins.

In the present specification, a device may be a microarray, beads, or a column, etc., having substances bound thereto (probes) capable of binding to one or more types, two or more types, three or more types, four or more types, or five types of marker RNAs. This device may be the one for measuring binding levels between the marker RNAs mentioned above and substances (probes) that specifically bind to the marker RNAs.

The "microarray" refers to a device for use in a method for quantifying one or more markers at once. Plural types of probes or antibodies or antigen binding fragments thereof that bind to a single marker may be bound to the microarray. For example, full-length cDNA complementary to the marker RNA or a cDNA fragment that hybridizes to a portion of the marker RNA as a probe may be bound to a DNA microarray.

### 5. Method for measuring marker protein

In an alternative aspect, the present invention relates to a method for determining a level of at least one type of protein selected from COPB2, KRAS, PRKCB, RHOC, CD147, CAPN2, ECM1, FGG, MFAP4, ADI1, AK1, MGAT1, CLDN3, CRP, UQCRC2, FGA, FGB, FGL1, GPX1, GSK3B, LBP, PDGFC, RAB13, RAP1B, SLC6A4, UBA7, ORM1, RNPEP, ANGPT1, APOB, B4GALT1, BHMT, CPN1, GNAZ, ICAM2, SELL, MAN1A1, SERPINA5, PACSIN2, NCF1B, TMEM59, YWHAB, ABAT, ADH1B, ASL, ASS1, CDH2, CAB39, CPS1, CD226, COL6A3, CUL4A, DSC1, ENTPD5, EIF4A1, FN1, PGC, RHEB, GNAI2, GNB1, GNA13, ITGA2B, ITGB1, ILK, F11R, LTA4H, LIMS1, NAV2, FAM129B, NNMT, NID1, PPIA, PLA1A, PPBP, PECAM1, GP1BB, PCSK9, MENT, SERPINA10, F2RL3, LOX, SFTPB, RAB5B, RALB, REEP6, RETN, AGXT, CCT2, THBD, ISG15, and ZYX contained in exosomes derived from blood derived from a patient, comprising
contacting a protein contained in the exosomes derived from blood derived from the patient with the composition or the device.

More specifically, the present invention may provide a method for measuring a level of one or more types of marker protein contained in exosomes derived from blood derived from a patient, comprising:
contacting a protein contained in the exosomes derived from blood derived from the patient with the composition or the device;
measuring a binding level of the marker protein in the exosomes bound to the substance capable of binding to the marker protein in the composition or the device; and
determining the marker protein level in the exosomes from the measured binding level.

In an alternative aspect, the present invention relates to a method for measuring a level of one or more types of marker protein contained in exosomes derived from blood derived from a patient, and a level of one or more types of marker RNA contained in blood derived from the patient, comprising:
contacting a protein contained in the exosomes derived from blood derived from the patient with the composition or the device; and
contacting RNA in blood derived from the patient with the composition or the device.

More specifically, the present invention may provide a method for measuring a level of one or more types of marker protein contained in exosomes derived from blood derived from a patient, and a level of one or more types of marker RNA contained in blood derived from the patient, comprising:
contacting a protein contained in the exosomes derived from blood derived from the patient with the composition or the device having a substance capable of binding to the marker protein;
measuring a binding level of the marker protein in the exosomes bound to the substance capable of binding to the marker protein in the composition or the device; and
determining the marker protein level in the exosomes from the measured binding level, and
contacting RNA contained in a blood sample derived from the patient with the composition or the device having a probe capable of binding to the marker RNA;
measuring a binding level of the marker RNA in the blood bound to the probe in the composition or the device; and
determining the marker RNA level in the blood from the measured binding level.

Throughout the present specification, marker RNA or marker protein to be measured, or marker RNA or marker protein contained in a composition or a device may be one or more types, two or more types, three or more types, four or more types, or five or more types. In the case of measuring two or more types, a likelihood of developing severe symptom may be determined by taking all measurement results together into consideration. For example, in the case where all of the measured markers indicate determination that the patient is likely to develop severe symptom, the likelihood of developing severe symptom may be determined to be higher than the case where some of measured markers indicate determination that the patient is likely to develop severe symptom. Alternatively, the likelihood of developing severe symptom may be determined by weighting each marker and placing emphasis on results about marker RNA of high importance. For example, in results of Examples of the present application, proteins found to be more beneficial for the discrimination of patient developing severe symptom, such as proteins having high sensitivity and specificity (e.g., COPB2, KRAS, PRKCB, RHOC, and CD147) and proteins having a low P value (RHOC, ECM1, FGG, and MFAP4), can be regarded as being of high importance.

Throughout the present specification, a substance capable of binding to a marker RNA or a substance capable of binding to a marker protein, used in the method of the present invention or contained in the composition, the kit and the device of the present invention may be two or more types for one type of marker RNA or one type of marker protein. Accordingly, in the method of the present invention, for example, two or more types (three or more types, four or more types, etc.) of different probes capable of binding to the same marker RNA, or two or more types (three or more types, four or more types, etc.) of primers capable of binding to the same marker RNA may be used. In the method of the present invention, two or more types (three or more types, four or more types, etc.) of different antibodies or antigen binding fragments thereof capable of binding to the same marker protein may be used. The composition, the kit and the device of the present invention may comprise two or more types (three or more types, four or more types, etc.) of different probes capable of binding to the same marker RNA, or two or more types (three or more types, four or more types, etc.) of primers capable of binding to the same marker RNA. The composition, the kit and the device of the present invention may comprise two or more types (three or more types, four or more types, etc.) of different antibodies or antigen binding fragments thereof capable of binding to the same marker protein.

The invention of the present application may be the following invention.
(1) A testing method for use in predicting whether there is a likelihood that the test subject develops severe symptom of coronavirus infectious disease (COVID-19), the coronavirus testing method comprising the steps of:
   collecting a body fluid such as blood, nasal discharge, or saliva as a specimen from a test subject;
   recovering exosomes from the collected body fluid;
   extracting a protein from the recovered exosomes;
   analyzing the protein extracted from the exosomes to obtain protein information thereon (information on the type and expression level of the protein); and
   predicting whether there is a likelihood that the test subject develops severe symptom on the basis of the obtained protein information (information on the type and expression level of the protein).
(2) The coronavirus testing method according to (1), wherein any one of predetermined particular types of proteins of the proteins derived from the exosomes, or a combination of two or more of the predetermined particular types are used as a predictive marker (biomarker) for the development of severe symptom in order to predict whether there is a likelihood that the test subject develops severe symptom.
(3) The coronavirus testing method according to (1), wherein
   in the step of predicting a likelihood of developing severe symptom,
   a likelihood that the test subject develops severe symptom is predicted when the expression level of the predetermined particular type of protein exceeds a predetermined reference value.
(4) The coronavirus testing method according to (1), wherein
   the protein includes one or both of
   an exosomal membrane protein, and
   an intra-exosomal protein.
(5) The coronavirus testing method according to (1), wherein
   the test subject is
   an individual infected with coronavirus (SARS-CoV-2),
   an individual suspected of being infected with coronavirus (SARS-CoV-2), or
   an individual, the presence or absence of coronavirus (SARS-CoV-2) infection of which is unknown.
(6) A system for use in carrying out a coronavirus testing method according to any of (1) to (5), the coronavirus testing system comprising:
   a sampling unit for collecting a body fluid such as blood, nasal discharge, or saliva as a specimen from a test subject;
   a preservation unit for preserving the body fluid, such as blood, nasal discharge, or saliva, collected as a specimen;
   an exosomes recovery unit for recovering exosomes from the collected and preserved body fluid;
   a protein extraction unit for extracting a protein from the recovered exosomes;
   a protein analysis unit for analyzing a protein obtained from the protein derived from the exosomes to obtain protein information thereon (information on the type and expression level of the protein); and
   a severe symptom development prediction unit for predicting whether there is a likelihood that the test subject develops severe symptom on the basis of the obtained protein information (information on the type and expression level of the protein).
(7) A virus testing method using
   a user system having a communication function which is used by a user such as a virus test subject, a virally infected individual, a patient, a hospital, or public administration, and
   an analysis and determination system having a communication function which is used by a testing center in charge of the virus test, wherein
   the user system has:
      a sampling unit for collecting a body fluid such as blood, nasal discharge, or saliva as a specimen from a test subject;
      a preservation unit for preserving the body fluid, such as blood, nasal discharge, or saliva, collected as a specimen;
      a recovery unit for recovering exosomes from the collected and preserved body fluid;
      a step of extracting a protein from the recovered exosomes;
      a protein analysis unit for analyzing the protein contained in the recovered exosomes to obtain protein information thereon (information on the type and expression level of the protein); and
      a communication unit for communicating with the analysis and determination system via internet, and
   the analysis and determination system has:
      a severe symptom development prediction unit for predicting whether there is a likelihood that the test subject develops severe symptom on the basis of the protein information transmitted from the user system; and
      a communication unit for communicating with the user system via internet, the coronavirus testing method comprising:
         (a) a step in which the user system recovers exosomes from the body fluid collected from the test subject;
         (b) a step in which the user system extracts a protein from the recovered exosomes;
         (d) a step in which the user system analyzes the protein extracted from the exosomes to obtain protein information thereon (information on the type and expression level of the protein);
         (f) a step in which the user system transmits the obtained protein information to the analysis and determination system via internet;
         (g) a step in which the analysis and determination system predicts whether there is a likelihood that the test subject develops severe symptom on the basis of the protein information (information on the type and expression level of the protein) received from the user system; and
         (k) a step in which the analysis and determination system transmits information on the prediction results of the step (g) to the user system via internet.
(8) A virus testing method comprising the steps of:
   collecting a body fluid such as blood, nasal discharge, or saliva as a specimen;
   recovering exosomes from the collected body fluid;
   extracting a protein from the recovered exosomes;
   analyzing the protein extracted from the exosomes to obtain protein information;
   analyzing a type of coronavirus (SARS-CoV-2) on the basis of the protein information obtained from the exosomes;
   determining the presence or absence of coronavirus infection on the basis of the analyzed type of coronavirus; and
   determining severity of coronavirus infectious disease (COVID-19) at a plurality of stages when the presence of coronavirus infection is determined.
(9) A system for use in carrying out a virus testing method according to (8), the virus testing system comprising:
   a sampling unit for collecting a body fluid such as blood, nasal discharge, or saliva as a specimen;
   a preservation unit for preserving the body fluid, such as blood, nasal discharge, or saliva, collected as a specimen;
   an exosomes recovery unit for recovering exosomes from the collected and preserved body fluid;
   a protein extraction unit for extracting a protein from the recovered exosomes;
   a protein analysis unit for analyzing the protein contained in the recovered exosomes to obtain protein information;
   a virus analysis unit for analyzing a type of coronavirus (SARS-CoV-2) on the basis of the protein information obtained from the exosomes;
   a first determination unit for determining the presence or absence of coronavirus infection on the basis of the analyzed type of coronavirus; and
   a second determination unit for determining severity of coronavirus infectious disease (COVID-19) at a plurality of stages when the presence of coronavirus infection is determined.
(10) A virus testing method using
   a user system having a communication function which is used by a user such as a virus test subject, a virally infected individual, a patient, a hospital, or public administration, and
   an analysis and determination system having a communication function which is used by a testing center in charge of the virus test, wherein
   the user system has:
      a sampling unit for collecting a body fluid such as blood, nasal discharge, or saliva as a specimen;
      a preservation unit for preserving the body fluid, such as blood, nasal discharge, or saliva, collected as a specimen;
      a recovery unit for recovering exosomes from the collected and preserved body fluid;
      a step of extracting a protein from the recovered exosomes;
      a protein analysis unit for analyzing the protein contained in the recovered exosomes to obtain protein information; and
      a communication unit for communicating with the analysis and determination system via internet, and
   the analysis and determination system has:
      a virus analysis unit for analyzing a type of coronavirus (SARS-CoV-2) on the basis of the protein information transmitted from the user system;
      a first determination unit for determining the presence or absence of coronavirus infection on the basis of the analyzed type of coronavirus;
      a second determination unit for determining severity of coronavirus infectious disease (COVID-19) at a plurality of stages when the presence of coronavirus infection is determined; and
      a communication unit for communicating with the user system via internet, the virus testing method comprising:
         (a) a step in which the user system recovers exosomes from the body fluid collected from a virus test subject, a virally infected individual, or a patient;
         (b) a step in which the user system extracts a protein from the recovered exosomes;
         (d) a step in which the user system analyzes the protein extracted from the exosomes to obtain protein information;
         (f) a step in which the user system transmits the protein information obtained from the exosomes to the analysis and determination system via internet;
         (g) a step in which the analysis and determination system analyzes a type of coronavirus (SARS-CoV-2) on the basis of the protein information received from the user system;
         (h) a step in which the analysis and determination system determines the presence or absence of coronavirus infection on the basis of the analyzed type of coronavirus;
         (i) a step in which the analysis and determination system determines severity of coronavirus infectious disease (COVID-19) at a plurality of stages when the presence of coronavirus infection is determined.
   (j) a step in which the analysis and determination system transmits information on the determination results of the step (h) to the user system via internet; and (k) a step in which the analysis and determination system transmits information on the determination results of the step (i) to the user system via internet.
(11) A coronavirus testing method comprising collecting and preserving a body fluid such as blood, nasal discharge, or saliva as a specimen, extracting an exosomal protein from this body fluid, and instantly analyzing a type of coronavirus (SARS-CoV-2) on the basis of this protein information to determine not only the presence or absence of coronavirus infection but whether the infection ends with mild symptom or whether severe symptom is developed.
(12) An apparatus for carrying out a coronavirus testing method according to (11), comprising: a testing apparatus comprising a sampler which collects and preserves a body fluid such as blood, nasal discharge, or saliva as a specimen, and a measurement device which extracts an exosomal protein from this body fluid; and an analysis apparatus with an analyzer which instantly determines a type of coronavirus on the basis of this protein information, in order to determine not only the presence or absence of coronavirus infection but whether the infection is with a mild symptom or whether a severe symptom is developed.
(13) The coronavirus testing method according to (10) which is a home-based testing method for coronavirus, comprising computerizing and transmitting specimen information from a user such as a patient, a hospital, or public administration to a testing site such as a testing center on the basis of a communication unit, such as a personal computer, established under an agreement between the user and the testing site, and computerizing and reporting information on results of instantly analyzing the specimen information from the testing site to the user, in order to determine not only the presence or absence of coronavirus infection but whether the infection ends with a mild symptom or whether a severe symptom is developed.

First, an apparatus related to the test and analysis of new coronavirus comprises a testing instrument and an analysis instrument.

The testing instrument refers to a sampler and a measurement device.

The sampler of the former collects and preserves a body fluid such as blood as a specimen. This varies depending on the type of the specimen. Any container or reagent may be a commercially available product.

The measurement device of the latter extracts an exosomal protein from the body fluid collected as a specimen.

The analysis apparatus employs a system that instantly determines a type of new coronavirus according to deep learning on the basis of information on testing results.

Second, a testing method for new coronavirus involves collecting a body fluid as a specimen with the sampler and preserving the body fluid. An exosomal protein is extracted from this body fluid with the measurement device. A type of new coronavirus is instantly determined according to deep learning with the analyzer on the basis of information on testing results.

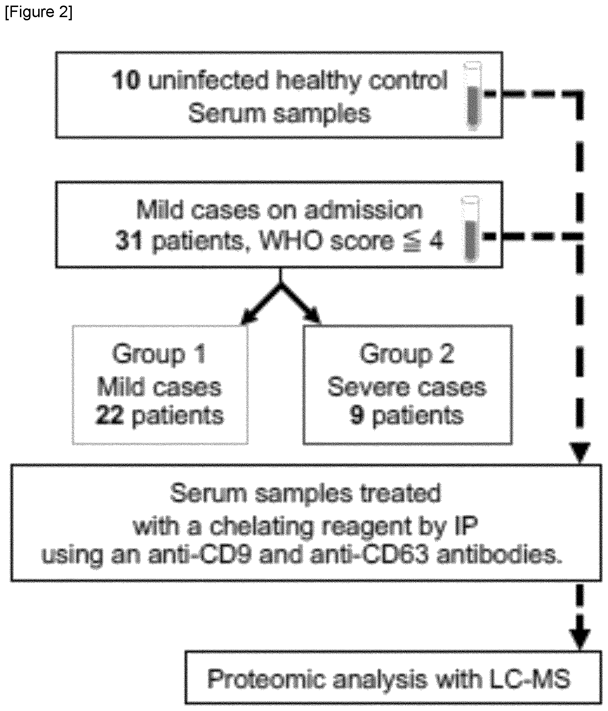

Third, a home-based testing method (19) for new coronavirus is as described in Figure 2. As for a user (14) and a testing site (13), the user (14) requests a test for new coronavirus and pays for it, while the testing site (14) consents to the acceptance of this test and agrees to the shipment of a testing apparatus (8). This agreement is made between the user and the testing site with communication units (15)(16) including personal computers as well as smartphones and televisions.

The user (14) collects (4) a body fluid as a specimen on the basis of operation instructions via a video, extracts (5) a protein from this body fluid with a testing apparatus (7), and computerizes (digitizes) and transmits (17) test information. By contrast, the testing site (13) analyzes the test information with an analysis apparatus (9) and computerizes and reports (18) information on the results to the user.

Hereinafter, the present invention will be described in more detail with reference to Examples. However, the scope of the present invention is not limited thereby. Literatures cited throughout the specification of the present application are incorporated in the specification of the present application by reference in their entirety.

### (Example 1) Patient group and clinical parameter influencing development of severe symptom

42 SARS-CoV-2-positive patients who visited the Jikei University Hospital from March to May of 2020 were enrolled (approved by the review committee of the Jikei University (number: 32-055 (10130))). Patient having SARS-CoV-2 RNA positivity determined by PCR tests using nasopharyngeal swabs were regarded as COVID-19 (SARS-CoV-2 infection) patient. A severity of the patient was determined on the basis of WHO 2020 Scoring shown in Table 2. 31 COVID-19 patients having a mild status (WHO score = 3) at the time of enrollment were enrolled, and 11 patients with severe symptom were excluded. All the COVID-19 patient received standard treatment (involving no steroid) based on the WHO living guidance for clinical management of COVID-19. The healthy persons tested were ten persons (approved by the institutional review committee of the Institute of Medical Science, the University Of Tokyo (number: 28-19-0907)) of age cohorts for the COVID-19 patient among healthy donors who visited Omiya City Clinic (Saitama City, Saitama, Japan) for regular health checkup from March to April of 2019.

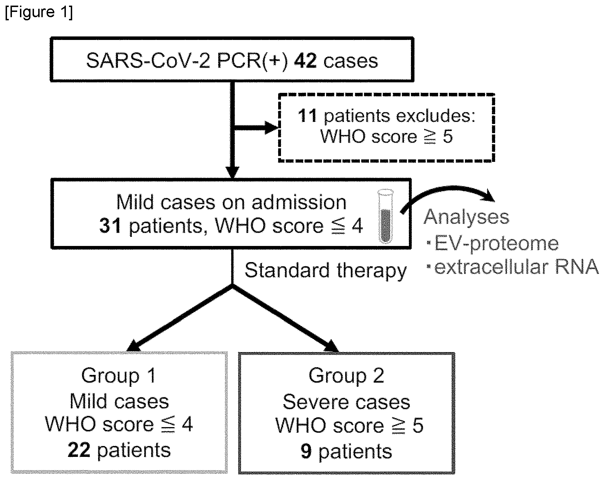

Blood obtained from each test subject at the time of enrollment was centrifuged at 3000 rpm at 4°C for 10 minutes to collect a serum sample as a supernatant, which was then preserved at -80°C. The correlation between each marker in the obtained serum sample and a clinical course after enrollment was retrospectively studied. As a result of dividing the 31 COVID-19 patients into the following two groups according to the degree of progression of disease from the course after enrollment (Figure 1), group 1 (mild case) involved 22 persons, and group 2 (severe case) involved 9 persons.
Group 1 (mild case): patients who maintained a mild status (WHO score ≤ 4)
Group 2 (severe case): patients who progressed to a severe status (WHO score ≥ 5)

Two of the patients of group 2 died of COVID-19 complications. Table 3 shows the backgrounds and the progression of symptom after sample collection of the individual patient. Table 4 shows statistical values of clinical parameters. No difference in age, sex, BMI, smoking index, blood urea nitrogen (BUN), creatinine (Cr), alanine aminotransferase (ALT), hypertension history, diabetes mellitus, dyslipidemia, and coronary heart disease was seen between the healthy person and the COVID-19 patient (P > 0.05). On the other hand, significant difference was confirmed in white blood cell (WBC) counts and C-reactive protein (CRP) values (P < 0.05). No difference in sex, BMI, WBC counts, BUN, Cr, creatinine kinase (CK), D-dimer, fibrinogen, hypertension history, diabetes mellitus, dyslipidemia, and coronary heart disease was seen between the COVID-19 patient of group 1 and group 2 (P > 0.05). On the other hand, significant difference was confirmed in age, smoking index, CRP value, and ALT value (P < 0.05). Accordingly, four parameters, age, smoking index, CRP values, and ALT values, were found to be associated with the development of severe symptom.

**[Table 2]**

| Patient | Descriptor | Score |
|---|---|---|
| Uninfected | No clinical or virological evidence of infection | 0 |
| Ambulatory | No limitation of activities | 1 |
| | Limitation of activities | 2 |
| Hospitalized-mild disease | No oxygen therapy | 3 |
| | Oxygen by nasal prongs or mask | 4 |
| Hospitalized-severe disease | Non-invasive ventilation of high-flow oxygen | 5 |
| | Intubation and mechanical ventilation | 6 |
| | Ventilation + additional organ support (pressors, RRT, ECMO) | 7 |

| | | |
|---|---|---|
| RRT: renal replacement therapy, ECMO: extracorporeal membrane oxygenation. | | |

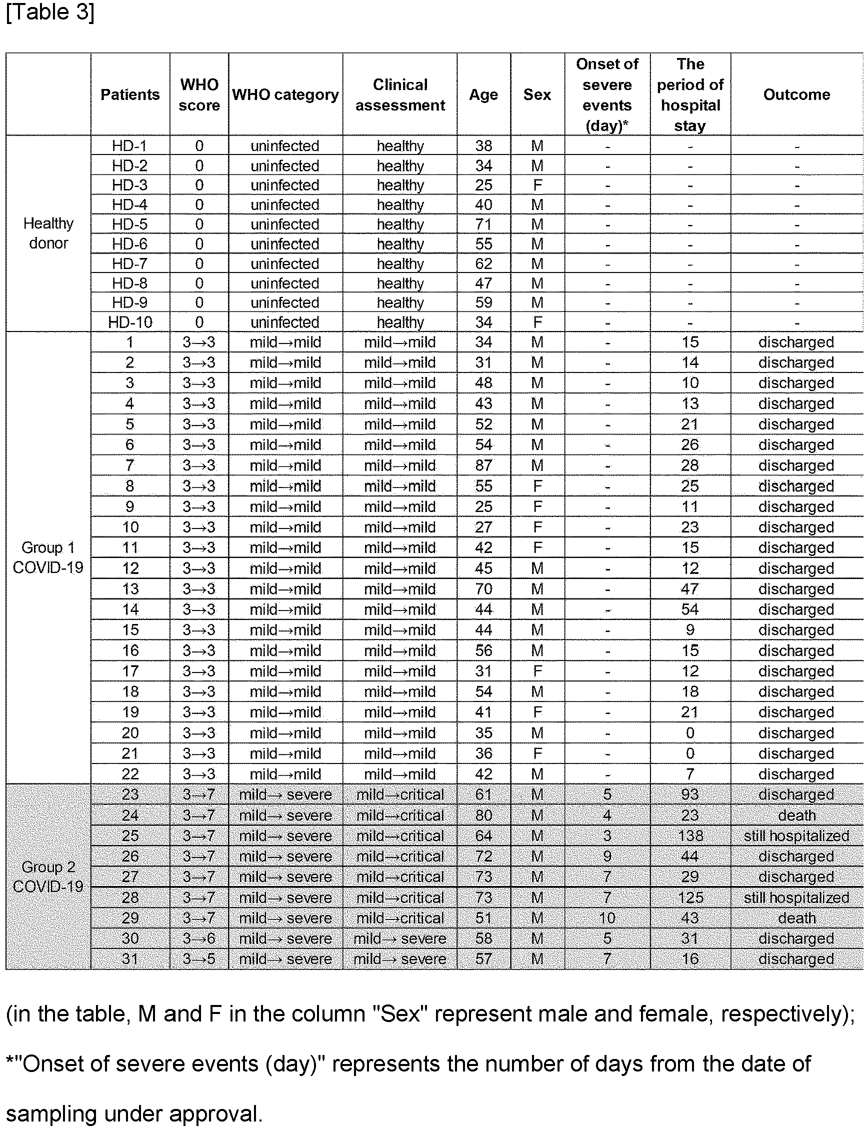

**[Table 4]**

| **Table 2. Differences of characteristics among COVID-19 patients and healthy donors.** | | | | | | |
|---|---|---|---|---|---|---|
| | | | | | | |

| | | uninfected | COVID-19 | | P value | |
|---|---|---|---|---|---|---|
| | | | Group 1 | Group 2 | | |
| | | (n=10) | (n=22) | (n=9) | (uninfected vs. infected) | (Group 1 vs. Group 2) |
| Aqe (year) | | 46.5 ± 14.8 | 45.3 ± 14.3 | 65.4 ± 9.5 | 0.42 | 0.001 |
| Sex | Men | 8 (80%) | 15 (68.2%) | 9 (100%) | 1.00 | 0.08 |
| | Women | 2 (20%) | 7 (31.8%) | 0 (0%) | | |
| BMI (kg/m²) | | 22.8 1 4.4 | 23.0 ± 3.0 (n=20) | 22.4 ± 3.2 | 0.95 | 0.63 |
| Smoking index | | 176 ± 237 | 60 ± 140 | 427 ± 670 | 0.94 | 0.02 |
| WBC count (10³/uL) | | 6.7 ± 2.9 | 4.6 ± 1.5 | 5.2 ± 3.2 | 0.03 | 0.46 |
| CRP (mg/dL) | | 0.12 ± 0.12 | 2.50 ± 2.41 | 6.07 ±5.11 | 0.01 | 0.01 |
| BUN (mg/dL) | | 13.7 ± 4.5 | 17.0 ± 16.6 | 22.6 ± 14.6 | 0.35 | 0.39 |
| Cr (mg/dL) | | 0.84 ± 0.13 | 1.72 ± 3.22 | 1.88 ± 2.4 | 0.33 | 0.90 |
| ALT (IU/L) | | 22.5 ± 8.5 | 21.4 ± 15.2 | 42.6 ± 33.2 | 0.51 | 0.02 |
| CK (IU/L) | | NA | 69.5 ± 44.1 | 71.8 ± 50.8 | NA | 0.90 |
| D-Dimer (µg/mL) | | NA | 1.8 ± 2.0 (n=12) | 1.1 ± 0.4 | NA | 0.28 |
| Fbg (mg/dL) | | NA | 389.0 ± 84.2 (n=7) | 480.4 ± 172.2 | NA | 0.22 |
| Hypertension | | 2 (20.0%) | 6 (27.3%) | 3 (33.3%) | 0.70 | 1.00 |
| Diabetes mellitus | | 0 (0%) | 5 (22.7%) | 3 (33.3%) | 0.17 | 0.66 |
| Dyslipidemia | | 0 (0%) | 4(18.2%) | 3 (33.3%) | 0.16 | 0.38 |
| Colonarv heart disease | | 0 (0%) | 2(9.1%) | 0 (0%) | 1.00 | 1.00 |
| | | | | | | |
| Continuous variables were expressed as mean ± SD and tested by Student's *t*-test. | | | | | | |
| Categorical variables were expressed as n (%) and tested by Fisher's exact test. | | | | | | |

### (Example 2) Measurement of protein in exosomes (EV)

### (1) Isolation of exosomes (EV)

An anti-CD9 antibody and an anti-CD63 antibody (HU Group Research Institute, Tokyo) attached to Dynabeads M-280 Tosylactivate (Thermo Fisher Scientific Inc. Waltham, MA, USA) were treated with chelate-based PEVIA(R) reagent (HU Group Research Institute) and then incubated on a rotator at 4°C for 18 hours. The beads were washed three times with PBS and further preserved at 4°C until analysis.

### (2) Preparation of peptide

The obtained exosomes (EV) were treated with S-Trap micro spin column (AMR Inc. Tokyo, Japan) according to a slight modification of manufacturer's instruction. Specifically, the exosomes were suspended in 50 µL of 50 mM TEAB buffer (Honeywell Inc. Charlotte, North Carolina, USA), pH 7.5, containing 5% SDS (FUJIFILM Wako Pure Chemical Corporation, Osaka, Japan). After removal of the beads, the amounts of proteins from EV were measured using MicroBCA(TM) protein assay kit (Thermo Fisher Scientific Inc.). 13.8 ng of Pierce(TM) digestion indicator for mass spectrometry (Thermo Fisher Scientific Inc.) was added to the lysate sample for the quality control of digestion efficiency.

### (3) Proteomic analysis by LC-MS

The peptides obtained from the EV proteins were reconstituted with 10 µl of water containing 0.1% formic acid (FA) (Fisher Chemical, Thermo Fisher Scientific Inc.). The peptides were quantified using Pierce(TM) Quantitative Fluorometric Peptide Assay (Thermo Fisher Scientific Inc.). The proteomic analysis of the peptides was carried out using Q Exactive (Thermo Fisher Scientific Inc.) loaded with UltiMate 3000 Nano LC Systems (Thermo Fisher Scientific Inc.). The peptide sample (1 µg) was injected using Dream Spray interface (AMR Inc.) to Acclaim PepMap 1000 trap column (75 µm × 2 cm, nanoViper C183 µm, 100 angstroms, Thermo Fisher Scientific Inc.) heated to 40°C in a chamber connected to C18 reverse-phase Aurora UHPLC emitter column equipped with nano Zero & Captive Spray Insert (75 µm × 25 cm, Ion Opticks Pty Ltd.). The flow rate of the nano pump was set to 250 nL/min in a gradient for 302 minutes, and the mobile phases used were A (0.1% FA in water, Fisher Chemical, Thermo Fisher Scientific Inc.) and B (0.1% FA in acetonitrile, Fisher Chemical, Thermo Fisher Scientific Inc.). The gradient of chromatography was designed so as to increase linearly from 2% B from 0 to 8 minutes and set to from 2% B to 35% B from 8 to 272 minutes, from 35% B to 70% B from 272 to 282 minutes, and from 70% B to 95% B from 282 to 283 minutes. Washing was performed for 8 minutes, and equilibration was performed for 10 minutes. The acquisition of data dependence was carried out in a cation mode. The parameters of mass spectrometry and the parameters of Proteome Discoverer 2.2.0.388 software (Thermo Fisher Scientific Inc.) were set according to the method described in the previous report (Ayako Kurimoto et al., Enhanced recovery of CD9-positive extracellular vesicles from human specimens by chelating reagent, doi: https://doi.org/10.1101/2020.06.17.155861).

### (Example 3) Measurement of serum miRNA

Total RNA was extracted from aliquots (200 µL) of the serum sample collected in Example 1 using QIAzol and miRNeasy Mini Kit (Qiagen N.V., Hilden, Germany) according to manufacturer's protocol. Libraries were prepared using QIAseq miRNA Library Kit (Qiagen N.V.). The prepared libraries were subjected to quality control using Bioanalyzer 2100 or TapeStation 4200 system (Agilent Technologies, Inc. Santa Clara, CA, USA). The library pools were quantified using Library Quantification Kit (Takara Bio Inc., Shiga, Japan) and sequenced using NovaSeq 6000 sequencing platform (Illumina Inc., San Diego, CA, USA). The determined sequences were pretreated using CLC Genomics Workbench v20.0.1 and annotated for miRBase v22.1 and Ensembl non-coding DNA database release 100.

### (Example 4) Statistical processing

Clinical data was compared between two groups by use of Fisher's exact test of category variables and unpaired Student's t-test of continuous variables. In order to identify biomarker candidates from EV proteins and exRNA, candidates present at different levels of P < 0.05 among three test subject groups (uninfected, COVID-19 group 1, and group 2) were first selected by use of one-way analysis of variance (ANOVA). Principal component analysis (PCA) was carried out for the selected candidates using Partek Genomics Suite 7.0 (Partek, Inc., St. Louis, Missouri, USA). Subsequently, candidates excellent in discriminability between group 1 and group 2 were selected on the basis of linear discriminant analysis using leave-one-out cross-validation. Then, ROC analysis was conducted using R version 3.6.3 (R Foundation for Statistics Computing, http://www.R-project.org), compute.es package version 0.2-2, hash package version 2.2.6.1, MASS package version 7.3-51.5, mutoss package version 0.1-12, and pROC package version 1.16.2. The optimum cutoff value of each candidate was established on the basis of the maximum total score of sensitivity and specificity (Youden index). Prediction sensitivity, specificity, and accuracy were calculated using the cutoff value corresponding to each candidate. Kaplan-Meier analysis based on the log-rank test and Cox regression analysis were carried out using IBM SPSS Statistics 25 (IBM Japan, Tokyo, Japan). Correlation plots were generated using R version 3.6.3 and corrplot package version 0.84, and non-supervised hierarchical clustering analysis was carried out using Partek Genomics Suite 7.0. The limitations of statistical significance in every analysis were defined as two-tailed P values of 0.05.

### (Results)

### (1) LC-MS analysis of proteomic profile from EV in serum samples of COVID-19 patient and uninfected control

In clinical practice, the analysis of EV from liquid biopsies has received attention as a potential approach of providing diagnostic and prognostic biomarkers for various diseases. However, this strategy has not yet been widely used because of the absence of a standardized method for separating EV from patient. As a result of conducting various studies on the optimum method for preparing EV, an immunoprecipitation (IP)-based method targeting surface marker proteins of EV achieved rapid and specific separation. Specifically, the recovery of CD9+ or CD63+ positive EV from a serum sample by use of IP in the presence of a chelating reagent improved yields and purity and was suitable for subsequent EV proteomic analysis by LC-MS (Figure 2).

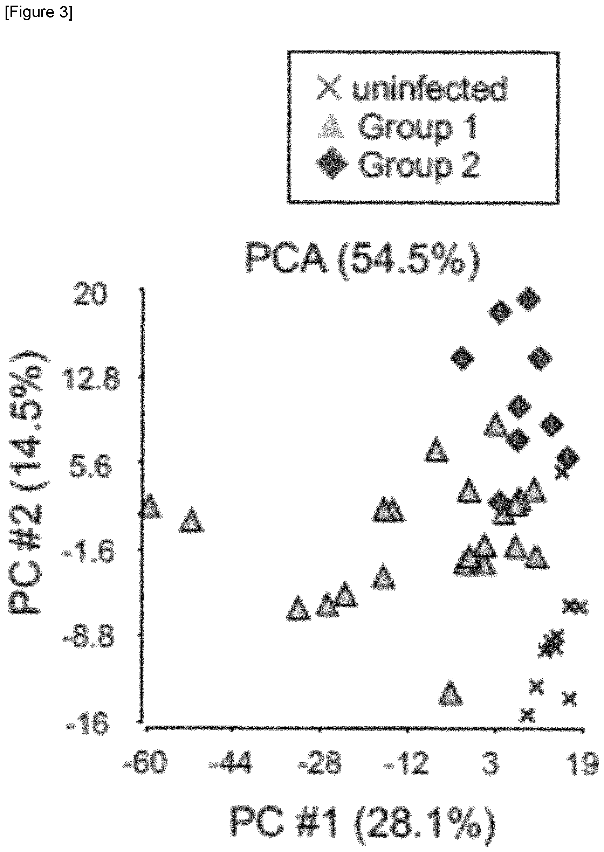

Proteins absent in all samples were excluded from 41 types of serum samples by LC-MS analysis. As a result, 1676 types of proteins were identified. Among these 1676 types of proteins, 723 types of proteins were present at different levels among three groups (P < 0.05; one-way analysis of variance). Non-supervised multivariate statistics based on principal component analysis (PCA) mapping was used to compare the expression patterns of these 723 types of EV-derived proteins among three patient cohorts. PCA plots first principal components (PC1) versus second principal components (PC2) using all term frequencies of LC-MS data and shows a trend of separation into three groups (Figure 3). PC1 (#1) accounted for 28.1% of variance, and PC2 (#2) accounted for 14.5% of variance, showing a trend in which the two principal components were able to achieve separation into three groups at a contribution rate of 54.5%.

These observation results indicated that the serum EV proteomic profiles of the COVID-19 patient deviated considerably from those of the uninfected test subjects (healthy persons). Although there was slight overlap or dispersion, evident difference in EV proteomics between group 1 and group 2 was found by the PCA score plot. In order to further identify the difference in EV-derived proteins between the patients of group 1 and group 2, cross-validation scores (Urabe F et al., Clin Cancer Res. 2019; 25 (10): 3016-25), which indicated the robustness of discrimination performance therebetween, were calculated on the basis of Fisher's linear discriminant analysis of each selected protein. 91 types of proteins having a cross-validation score exceeding 0.75 were listed from the candidate proteins (Tables 5 to 7).

**[Table 5]**

| EV proteins for discrimination between mild and severe COVID-19 patients. | | | | | |
|---|---|---|---|---|---|
| GeneName | Cross-validation score | Sensitivity | Specificity | Accuracy | AUC |
| COPB2 | 1.00 | 1.00 | 1.00 | 1.00 | 1.00 |
| KRAS | 0.90 | 1.00 | 0.82 | 0.87 | 0.93 |
| PRKCB | 0.90 | 0.89 | 0.95 | 0.94 | 0.93 |
| RHOC | 0.90 | 0.78 | 1.00 | 0.94 | 0.96 |
| CD147 | 0.87 | 0.67 | 0.91 | 0.84 | 0.73 |
| CAPN2 | 0.87 | 0.89 | 0.77 | 0.81 | 0.84 |
| ECM1 | 0.87 | 0.67 | 0.95 | 0.87 | 0.82 |
| FGG | 0.87 | 0.67 | 1.00 | 0.90 | 0.87 |
| MFAP4 | 0.87 | 0.56 | 1.00 | 0.87 | 0.75 |
| ADI1 | 0.84 | 0.67 | 0.95 | 0.87 | 0.88 |
| AK1 | 0.84 | 0.89 | 0.91 | 0.90 | 0.95 |
| MGAT1 | 0.84 | 1.00 | 0.77 | 0.84 | 0.91 |
| CLDN3 | 0.84 | 0.89 | 0.82 | 0.84 | 0.86 |
| CRP | 0.84 | 0.78 | 0.77 | 0.77 | 0.82 |
| UQCRC2 | 0.84 | 0.78 | 0.82 | 0.81 | 0.77 |
| FGA | 0.84 | 0.67 | 0.95 | 0.87 | 0.88 |
| FGB | 0.84 | 0.67 | 1.00 | 0.90 | 0.84 |
| FGL1 | 0.84 | 0.78 | 0.82 | 0.81 | 0.85 |
| GPX1 | 0.84 | 0.67 | 0.91 | 0.84 | 0.81 |
| GSK3B | 0.84 | 0.44 | 1.00 | 0.84 | 0.73 |
| LBP | 0.84 | 0.78 | 0.86 | 0.84 | 0.82 |
| PDGFC | 0.84 | 0.89 | 0.77 | 0.81 | 0.86 |
| RAB13 | 0.84 | 0.78 | 0.86 | 0.84 | 0.85 |
| RAP1B | 0.84 | 0.67 | 1.00 | 0.90 | 0.91 |
| SLC6A4 | 0.84 | 0.89 | 0.82 | 0.84 | 0.90 |
| UBA7 | 0.84 | 0.78 | 0.86 | 0.84 | 0.83 |
| ORM1 | 0.81 | 0.89 | 0.59 | 0.68 | 0.80 |
| RNPEP | 0.81 | 0.56 | 0.95 | 0.84 | 0.68 |
| ANGPT1 | 0.81 | 0.78 | 0.86 | 0.84 | 0.88 |
| APOB | 0.81 | 0.78 | 0.86 | 0.84 | 0.79 |

**[Table 6]**

| **GeneName** | **Cross-validation score** | **Sensitivity** | **Specificity** | **Accuracy** | **AUC** |
|---|---|---|---|---|---|
| BHMT | 0.81 | 0.44 | 1.00 | 0.84 | 0.74 |
| CPN1 | 0.81 | 0.89 | 0.68 | 0.74 | 0.84 |
| GNAZ | 0.81 | 1.00 | 0.95 | 0.97 | 0.99 |
| ICAM2 | 0.81 | 1.00 | 0.59 | 0.71 | 0.83 |
| SELL | 0.81 | 0.67 | 0.91 | 0.84 | 0.74 |
| MAN1A1 | 0.81 | 0.78 | 0.82 | 0.81 | 0.85 |
| SERPINA5 | 0.81 | 0.89 | 0.82 | 0.84 | 0.81 |
| PACSIN2 | 0.81 | 0.89 | 0.86 | 0.87 | 0.90 |
| NCF1B | 0.81 | 0.89 | 0.55 | 0.65 | 0.73 |
| TMEM59 | 0.81 | 0.44 | 0.95 | 0.81 | 0.59 |
| YWHAB | 0.77 | 0.67 | 0.95 | 0.87 | 0.85 |
| ABAT | 0.77 | 0.33 | 1.00 | 0.81 | 0.52 |
| ADH1B | 0.77 | 0.67 | 0.95 | 0.87 | 0.79 |
| ASL | 0.77 | 0.67 | 0.91 | 0.84 | 0.80 |
| ASS1 | 0.77 | 0.78 | 0.86 | 0.84 | 0.79 |
| CDH2 | 0.77 | 0.56 | 0.95 | 0.84 | 0.69 |
| CAB39 | 0.77 | 0.89 | 0.91 | 0.90 | 0.94 |
| CPS1 | 0.77 | 0.67 | 0.82 | 0.77 | 0.74 |
| CD226 | 0.77 | 0.67 | 1.00 | 0.90 | 0.87 |
| COL6A3 | 0.77 | 0.67 | 0.86 | 0.81 | 0.82 |
| CUL4A | 0.77 | 0.78 | 0.64 | 0.68 | 0.75 |
| DSC1 | 0.77 | 0.44 | 0.95 | 0.81 | 0.58 |
| ENTPD5 | 0.77 | 1.00 | 0.64 | 0.74 | 0.86 |
| EIF4A1 | 0.77 | 0.67 | 0.86 | 0.81 | 0.80 |
| FN1 | 0.77 | 0.89 | 0.68 | 0.74 | 0.81 |
| PGC | 0.77 | 0.89 | 0.68 | 0.74 | 0.78 |
| RHEB | 0.77 | 1.00 | 0.59 | 0.71 | 0.84 |
| GNAI2 | 0.77 | 0.89 | 0.59 | 0.68 | 0.79 |
| GNB1 | 0.77 | 0.78 | 0.77 | 0.77 | 0.83 |
| GNA13 | 0.77 | 0.67 | 0.95 | 0.87 | 0.86 |
| ITGA2B | 0.77 | 0.67 | 0.91 | 0.84 | 0.86 |
| ITGB1 | 0.77 | 1.00 | 0.59 | 0.71 | 0.84 |

**[Table 7]**

| **GeneName** | **Cross-validation score** | **Sensitivity** | **Specificity** | **Accuracy** | **AUC** |
|---|---|---|---|---|---|
| ITGB1 | 0.77 | 1.00 | 0.59 | 0.71 | 0.84 |
| ILK | 0.77 | 0.89 | 0.77 | 0.81 | 0.84 |
| F11R | 0.77 | 1.00 | 0.50 | 0.65 | 0.82 |
| LTA4H | 0.77 | 0.56 | 0.91 | 0.81 | 0.67 |
| LIMS1 | 0.77 | 0.89 | 0.77 | 0.81 | 0.83 |
| NAV2 | 0.77 | 0.56 | 0.86 | 0.77 | 0.73 |
| FAM129B | 0.77 | 0.78 | 0.86 | 0.84 | 0.84 |
| NNMT | 0.77 | 0.67 | 0.91 | 0.84 | 0.69 |
| NID1 | 0.77 | 0.89 | 0.55 | 0.65 | 0.76 |
| PPIA | 0.77 | 0.78 | 0.77 | 0.77 | 0.86 |
| PLA1A | 0.77 | 0.67 | 0.91 | 0.84 | 0.80 |
| PPBP | 0.77 | 0.67 | 0.77 | 0.74 | 0.69 |
| PECAM1 | 0.77 | 1.00 | 0.55 | 0.68 | 0.82 |
| GP1BB | 0.77 | 0.67 | 0.86 | 0.81 | 0.81 |
| PCSK9 | 0.77 | 1.00 | 0.82 | 0.87 | 0.91 |
| MENT | 0.77 | 0.44 | 0.95 | 0.81 | 0.71 |
| SERPINA10 | 0.77 | 0.67 | 0.82 | 0.77 | 0.74 |
| F2RL3 | 0.77 | 0.89 | 0.86 | 0.87 | 0.91 |
| LOX | 0.77 | 0.67 | 0.91 | 0.84 | 0.81 |
| SFTPB | 0.77 | 0.67 | 0.77 | 0.74 | 0.78 |
| RAB5B | 0.77 | 0.78 | 0.86 | 0.84 | 0.84 |
| RALB | 0.77 | 0.89 | 0.68 | 0.74 | 0.82 |
| REEP6 | 0.77 | 0.78 | 0.68 | 0.71 | 0.71 |
| RETN | 0.77 | 0.67 | 0.73 | 0.71 | 0.69 |
| AGXT | 0.77 | 0.22 | 1.00 | 0.77 | 0.48 |
| CCT2 | 0.77 | 0.89 | 0.64 | 0.71 | 0.81 |
| THBD | 0.77 | 0.56 | 0.91 | 0.81 | 0.74 |
| ISG15 | 0.77 | 0.33 | 1.00 | 0.81 | 0.67 |
| ZYX | 0.77 | 1.00 | 0.55 | 0.68 | 0.82 |

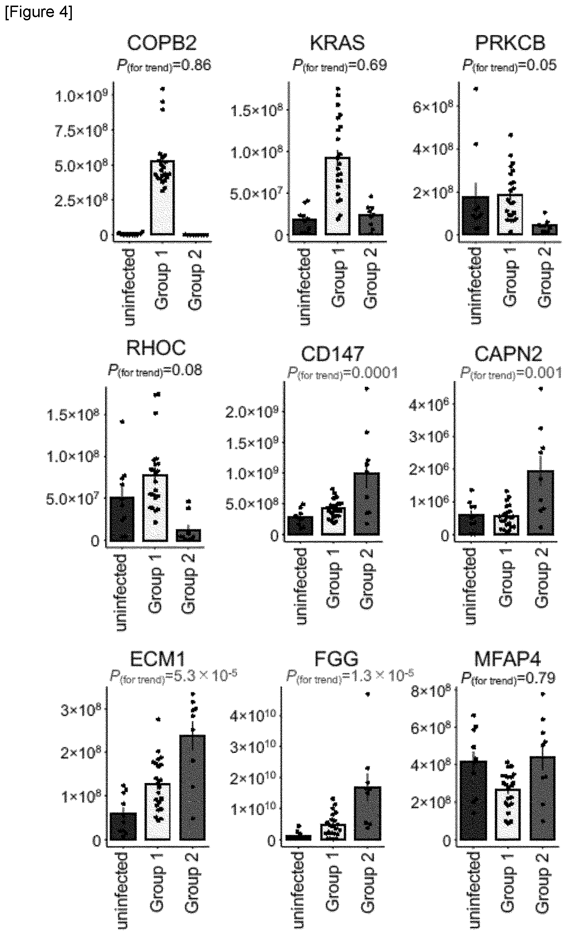

Figure 4 shows the abundances, in each group, of top nine proteins having a cross-validation score exceeding 0.85 among three patient groups. Among these proteins, four proteins, COPI coat complex subunit beta 2 (COPB2), KRAS proto-oncogene (KRAS), protein kinase C beta (PRKCB), and Ras homolog family member C (RHOC), exhibited a larger level in group 1 than in group 2 or uninfected controls (P trend > 0.05). Furthermore, the amounts of CD147, calpain-2 (CAPN2), extracellular matrix protein 1 (ECM1), and fibrinogen gamma chain (FGG) were significantly larger in group 2 than in uninfected controls or group 1 (P trend < 0.05). Only the amount of microfibril-associated protein 4 (MFAP4) was smaller in group 1 than in uninfected controls or group 2 (P trend > 0.05).

### (2) Predictive values of nine EV proteins for COVID-19 severity

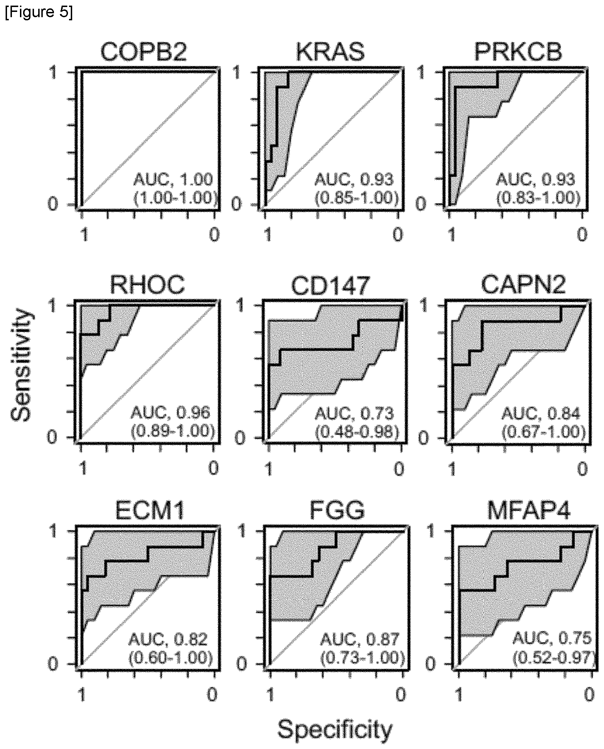

ROC analysis was carried out by using group 1 and group 2 in combination, and the robust test of sensitivity, specificity, and AUC was conducted for a set of nine predictive markers (Figure 5). The AUC values of four markers, COPB2, KRAS, PRKCM, and RHOC, whose abundance was increased in group 1 were 1.00 (95% CI: 1.00-1.00), 0.93 (95% CI: 0.85-1.00), 0.93 (95% CI: 0.83-1.00), and 0.96 (95% CI: 0.89-1.00), respectively. The AUC values of the other five markers, CD147, CAPN2, ECM1, FGG, and MFAP4, were 0.73 (95% CI: 0.48-0.98), 0.84 (95% CI: 0.67-1.00), 0.82 (95% CI: 0.60-1.00), 0.87 (95% CI: 0.73-1.00), and 0.75 (95% CI: 0.52-0.97), respectively.

These results suggested that this series of markers tested on admission of the patient are capable of significantly separating patients into those who develop mild COVID-19 and those who develop serious COVID-19. In order to identify the optimum cutoff values of the nine marker proteins according to the Youden index, additional ROC curves were generated.

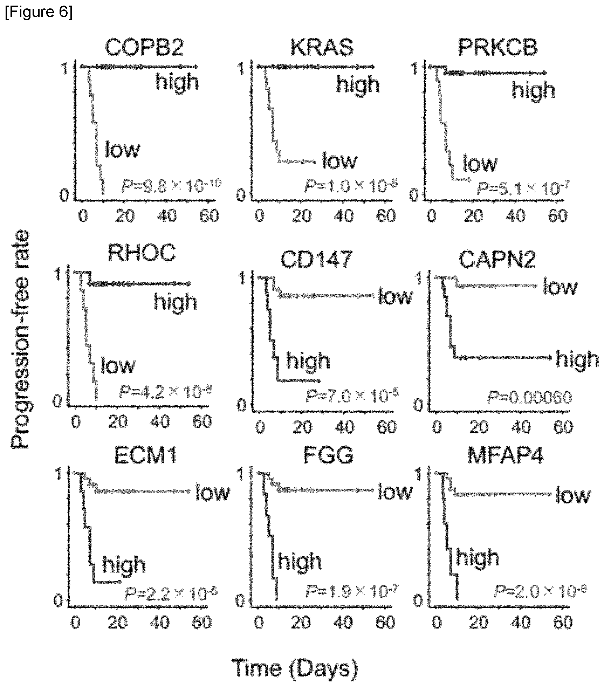

For further analysis, the COVID-19 patients were divided into low and high groups using the cutoff values, between which the development of serious COVID-19-associated events was then compared. A Kaplan-Meier curve that showed the time to the onset of serious events was prepared for each of the nine proteins (Figure 6). The period without developing severe symptom (progression-free period) of a group having small amounts of the proteins COPB2, KRAS, PRKCM, and RHOC whose abundance was increased in group 1 was significantly shorter than that of a group having large amounts of these proteins (P = 9.8 × 10⁻¹⁰; P = 1.0 × 10⁻⁵; P = 5.1 × 10⁻⁷; and P = 4.2 × 10⁻⁸, respectively). Conversely, the period without severe symptom (progression-free period) of a group having large amounts of the proteins CD147, CAPN2, ECM1, FGG, and MFAP4 whose abundance was increased in group 2 was significantly shorter than that of a group having small amounts of these proteins (P = 7.0 × 10⁻⁵; P = 0.00060; P = 2.2 × 10⁻⁵; P = 1.9 × 10⁻⁷; and P = 2.0 × 10⁻⁶, respectively). These results indicate that this series of markers is useful in predicting the likelihood that patient experience serious COVID-19-associated events.

### (3) NGS measurement of exRNA profile in serum samples from COVID-19 patient and uninfected control

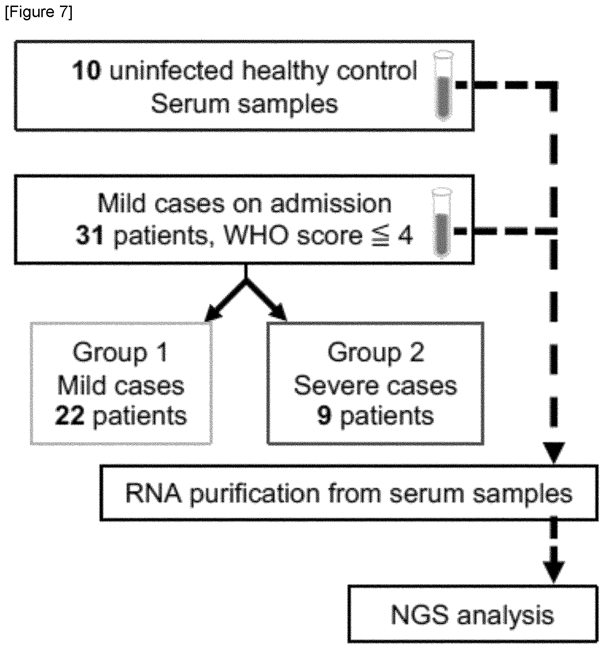

Circulating exRNA may function as a biomarker for a wide range of disease. ExRNA is constituted by diverse RNA subpopulations that are protected from degradation by uptake into EV or binding to lipids or proteins. ExRNA profiles in blood sample are dynamic and involve mRNA, miRNA, piRNA, and IncRNA (Murillo OD et al., Cell. (2019) 177 (2): 463-77 e15). In this Example, exRNA present in serum samples of patient was analyzed by use of next-generation sequencing (NGS) (Figure 7).

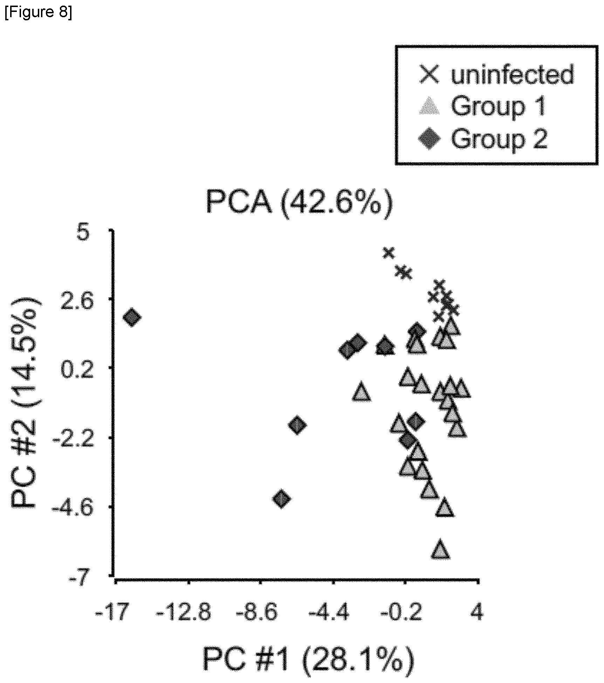

408 transcripts were identified from 41 types of serum samples by NGS analysis. However, transcripts having a readout of less than 50 in all the samples were excluded. Among these exRNAs, 43 transcripts were differentially expressed among three groups (P < 0.05; one-way analysis of variance). Non-supervised multivariate statistics based on PCA mapping was carried out to identify the expression patterns of these 43 transcripts among three groups. The PCA plot from NGS data revealed a trend of separation into three groups (Figure 8). This separation was explainable from first principal components (PS1) that accounted for 28.1% of variance. Second principal components (PS2) accounted for 14.5% of variance. These observation results indicated that the serum exRNA profiles of the COVID-19 patient deviated considerably from those of the uninfected donors. Although there was overlap or dispersion to some extent, evident difference in exRNA profile between group 1 and group 2 was able to be detected by the PCA plot.

In order to distinguish between group 1 and group 2, the cross-validation score of each selected transcript was calculated on the basis of Fisher's linear discriminant analysis. 14 transcripts having a cross-validation score exceeding 0.75 were selected from the candidate transcripts (Table 8).

**[Table 8]**

| **Transcript** | **Cross-validation score** | **Sensitivity** | **Specificity** | **Accuracy** | **AUC** |
|---|---|---|---|---|---|
| miR-122-5p | 0.84 | 0.56 | 1.00 | 0.87 | 0.81 |
| SNORD33 | 0.84 | 1.00 | 0.73 | 0.81 | 0.89 |
| AL732437.2 | 0.84 | 0.89 | 0.68 | 0.74 | 0.80 |
| RNU2-29P | 0.81 | 0.44 | 1.00 | 0.84 | 0.70 |
| CDKN2B-AS1 | 0.81 | 0.67 | 0.95 | 0.87 | 0.86 |
| AL365184.1 | 0.81 | 0.78 | 0.86 | 0.84 | 0.87 |
| AL365184.1 | 0.81 | 0.78 | 0.91 | 0.87 | 0.90 |
| AL365184.1 | 0.81 | 0.67 | 0.91 | 0.84 | 0.87 |
| AL365184.1 | 0.81 | 0.78 | 0.91 | 0.87 | 0.90 |
| AL365184.1 | 0.81 | 1.00 | 0.73 | 0.81 | 0.92 |
| let-7c-5p | 0.77 | 0.44 | 0.95 | 0.81 | 0.75 |
| miR-21-5p | 0.77 | 0.56 | 0.91 | 0.81 | 0.71 |
| miR-140-3p | 0.77 | 0.89 | 0.77 | 0.81 | 0.89 |
| C5orf66-AS2 | 0.77 | 0.78 | 0.91 | 0.87 | 0.89 |

### (4) Correlation among markers selected to predict severity value of disease

Next, the hazard ratios (HRs) of the exRNA markers and the EV protein markers were calculated by use of univariate Cox regression analysis. Particularly, HR of low COBP2 was unable to be statistically calculated using the optimum cutoff value, suggesting that EV COPB2 has the highest predictive value among the two sets of markers. High age (HR = 28.1; 95% CI = 3.4-231.9; P = 0.0019), high CRP (HR = 8.4; 95% CI = 1-67.5; P = 0.045), low PRKCB (HR = 32.1; 95% CI3.9-261.9; P = 0.0012), low RHOC (HR23.6;95%CI4.7-118;P=0.00012), high CD147 (HR10.7;95%CI2.5-45.1;P=0.0013), high CAPN2 (HR15.5;95%CI1.9-125.9;P=0.010), high ECM1 (HR11.6;95%CI2.8-48.4;P=0.00079), high FGG (HR21.4;95%CI4.2-110.4;P=0.00025), high MFAP4 (HR12.7;95%CI3.3-48.6;P=0.00022), high miR-122-5p (HR10.5;95%CI2.7-40.4;P=0.00063), high AL732437.2 (HR9.9;95%CI1.2-79.9;P=0.031), high RNU2-29P (HR10.4;95%CI2.6-40.8;P=0.00081), high CDKN2B-AS1 (HR14.4;95%CI3.4-61.3;P=0.00031), and high AL365184.1 (HR14.2;95%CI1.8-114.4;P=0.013) were statistically significant (Table 9).

**[Table 9]**

| | **Cut-off** | **HR** | **(95% CI)** | **P value** |
|---|---|---|---|---|
| Age high | 56.5 | 28.1 | (3.4 - 231.9) | 0.0019 |
| Smoking index high | 87.5 | 3.1 | (0.8 - 11.6) | 0.092 |
| CRP high | 2.3 | 8.4 | (1 - 67.5) | 0.045 |
| ALT high | 21.5 | 3.8 | (0.8 - 18.5) | 0.095 |
| COPB2 low | 1.6 x10⁸ | NA | - | - |
| KRAS low | 4.8 x10⁷ | 189.8 | (0.4 - 9.0 x10⁴) | 0.095 |
| PRKCB low | 6.3 x10⁷ | 32.1 | (3.9 - 261.9) | 0.0012 |
| RHOC low | 1.4 x10⁷ | 23.6 | (4.7 - 118) | 0.00012 |
| CD147 high | 6.1 x10⁸ | 10.7 | (2.5 - 45.1) | 0.0013 |
| CAPN2 high | 7.2 x10⁵ | 15.5 | (1.9 - 125.9) | 0.010 |
| ECM1 high | 2.3 x10⁸ | 11.6 | (2.8 - 48.4) | 0.00079 |
| FGG high | 1.4 x10¹⁰ | 21.4 | (4.2 - 110.4) | 0.00025 |
| MFAP4 high | 4.6 x10⁸ | 12.7 | (3.3 - 48.6) | 0.00022 |
| miR-122-5p high | 1.0 x10⁵ | 10.5 | (2.7 - 40.4) | 0.00063 |
| SNORD33 high | 406.6 | 104.1 | (0.4 - 2.7 x10⁴) | 0.10 |
| AL732437.2 high | 8.8 | 9.9 | (1.2 - 79.9) | 0.031 |
| RNU2-29P high | 124.1 | 10.4 | (2.6 - 40.8) | 0.00081 |
| CDKN2B-AS1 high | 6.2 | 14.4 | (3.4 - 61.3) | 0.00031 |
| AL365184.1 high | 2.9 | 14.2 | (1.8 - 114.4) | 0.013 |

| | | | | |
|---|---|---|---|---|
| HR, hazard ratio; CI, confidence interval; NA, not applicable | | | | |

In order to examine potential relationship among the selected markers, Spearman's correlation coefficients were calculated on the basis of marker levels. In order to visualize the correlation coefficients of 19 markers, a correlation plot was prepared (Figure 9). As a result, four hierarchical clusters of markers sharing strong positive correlation in groups to which they belonged were found. Each marker appeared to fit into one of the clearly defined four clusters (i.e., clusters 1, 2, 3, and 4). Particularly, cluster 1 (PRKCB, RHOC, COPB2, and KRAS) was in negative correlation with the other clusters, and clusters 2, 3, and 4 were substantially in strong positive correlation with each other.

All the four EV proteins of cluster 1 exhibited a significantly higher abundance in group 1 than in the COVID-19 patients of group 2. The level of MFAP4 did not significantly correlate with smoking or age. On the other hand, cluster 3 involved ECM1, CDKN2B.AS1, AL365184.1, CAPN2, CRP, FGG, and CD147. The levels of one exRNA (CDKN2B.AS1 of cluster 3) and four proteins associated with extracellular matrix formation (MFPA4 of cluster 2 and ECM1, CAPN2, and CD147 of cluster 3) correlated with the level of FGG, which plays an important role in coagulation (P < 0.05) (Figure 4). The levels of the markers of cluster 3 correlated with age associated with vascular endothelial dysfunction and coagulation (Donato AJ et al., Circ Res. 2018; 123 (7): 825-48). The great part of data suggested that clusters 2 and 3 represent groups of coagulation-associated markers. The components ALT, RNU2-29P, SNORD33, miR-122-5p, and AL732437.2 of cluster 4 probably reflect a phenomenon at least partially associated with hepatic damage. The level of ALT, which is typical transaminase associated mainly with hepatic function disorder, correlated with the levels of these three exRNA species (P < 0.05).

The results described above indicated that the EV protein and exRNA profiles in the serum of patient clearly reflect particular host response to the progression of SARS-CoV-2 infection and disease.

### Reference Signs List

13. Testing site
14. User
15. Central computer
16. Individualized computer
17. Transmission of information
18. Report of information
19. Home-based testing method for new coronavirus

## Claims

1. A method for determining a likelihood that a COVID-19 patient develops severe symptom, comprising:
measuring a level of one or more types of marker protein present in exosomes in blood derived from the patient; and
comparing the measured level of the marker protein with a control RNA level to determine the likelihood that the patient develops severe symptom, wherein
the marker protein is one or more types of protein selected from the following group: COPB2, KRAS, PRKCB, RHOC, CD147, CAPN2, ECM1, FGG, MFAP4, ADI1, AK1, MGAT1, CLDN3, CRP, UQCRC2, FGA, FGB, FGL1, GPX1, GSK3B, LBP, PDGFC, RAB13, RAP1B, SLC6A4, UBA7, ORM1, RNPEP, ANGPT1, APOB, B4GALT1, BHMT, CPN1, GNAZ, ICAM2, SELL, MAN1A1, SERPINA5, PACSIN2, NCF1B, TMEM59, YWHAB, ABAT, ADH1B, ASL, ASS1, CDH2, CAB39, CPS1, CD226, COL6A3, CUL4A, DSC1, ENTPD5, EIF4A1, FN1, PGC, RHEB, GNAI2, GNB1, GNA13, ITGA2B, ITGB1, ILK, F11R, LTA4H, LIMS1, NAV2, FAM129B, NNMT, NID1, PPIA, PLA1A, PPBP, PECAM1, GP1BB, PCSK9, MENT, SERPINA10, F2RL3, LOX, SFTPB, RAB5B, RALB, REEP6, RETN, AGXT, CCT2, THBD, ISG15, and ZYX.

2. The method according to claim 1, wherein the marker protein is one or more types of protein selected from the following group: COPB2, KRAS, PRKCB, RHOC, CD147, CAPN2, ECM1, FGG, and MFAP4, wherein
in the case where the measured protein is COPB2 or KRAS, the patient is determined to be likely not to develop severe symptom when the level of the protein is higher than that in a healthy person;
in the case where the measured protein is PRKCB or RHOC, the patient is determined to be likely to develop severe symptom when the level of the protein is lower than that in a healthy person or a mildly symptomatic patient;
in the case where the measured protein is any of CD147, CAPN2, ECM1, and FGG, the patient is determined to be likely to develop severe symptom when the level of the protein is higher than that in a healthy person or a mildly symptomatic patient; and
in the case where the measured protein is MFAP4, the patient is determined to be less likely to develop severe symptom when the level of the protein is lower than that in a healthy person.

3. The method according to claim 1 or 2, wherein the control protein level is a level of the marker RNA in a blood sample obtained from a healthy person or a COVID-19 patient who did not develop severe symptom early after infection.

4. The method according to any one of claims 1 to 3, wherein two or more types are used in combination.

5. The method according to claim 4, wherein the combination of the two or more types is the following combination (a) or (b):
(a) a combination of two or more types selected from PRKCB, RHOC, COPB2, and KRAS,
(b) a combination of two or more types selected from ECM1, CAPN2, FGG, and CD147.

6. The method according to any one of claims 1 to 5, further comprising
measuring one or more types of marker RNA level in blood derived from the patient, wherein
the likelihood of developing severe symptom is determined from the marker protein level and the marker RNA level in combination, wherein
the marker RNA is one or more types of RNA selected from the following group: miR-122-5p, SNORD33, AL732437.2, RNU2-29P, CDKN2B-AS1, AL365184.1, AL365184.1, AL365184.1, AL365184.1, AL365184.1, let-7c-5p, miR-21-5p, miR-140-3p, and C5orf66-AS2 and wherein
the measured level of the RNA higher than a control RNA level indicates that the patient is likely to develop severe symptom.

7. The method according to claim 6, wherein the marker RNA is selected from the group consisting of the following RNAs: miR-122-5p, SNORD33, AL732437.2, RNU2-29P, CDKN2B-AS1, and AL365184.1.

8. The method according to any one of claims 1 to 7, further comprising determining or measuring one or more types selected from age, smoking index, a CRP value in blood, and an ALT value in blood of the patient, wherein the likelihood of developing severe symptom is determined by using the age, the smoking index, the CRP value in blood, and/or the ALT value in blood in combination with the marker protein level or in combination with the marker protein level and the marker RNA level, wherein
the patient is determined to be likely to develop severe symptom when all of the age, the smoking index, the CRP, and the ALT have a higher numeric value than that of a control which is a healthy person or a mild case.

9. The method according to claim 8, comprising determining the development of severe symptom by a combination selected from the following (a) to (d):
(a) two or more factors selected from the group consisting of PRKCB, RHOC, COPB2, and KRAS,
(b) two or more factors selected from the group consisting of smoking index, age, and MFAP4,
(c) two or more factors selected from the group consisting of CDKN2B-AS1, AL365184.1, ECM1, CAPN2, CRP, FGG, and CD147, and
(d) two or more types of factors selected from the group consisting of ALT, RNU2-29P, SNORD33, miR-122-5p, and AL732437.2.

10. The method according to any one of claims 1 to 9, further comprising further subjecting a patient determined to be likely to develop severe symptom to control or treatment against the development of severe symptom.

11. A marker for severe COVID-19, which is at least one type of protein selected from COPB2, KRAS, PRKCB, RHOC, CD147, CAPN2, ECM1, FGG, MFAP4, ADI1, AK1, MGAT1, CLDN3, CRP, UQCRC2, FGA, FGB, FGL1, GPX1, GSK3B, LBP, PDGFC, RAB13, RAP1B, SLC6A4, UBA7, ORM1, RNPEP, ANGPT1, APOB, B4GALT1, BHMT, CPN1, GNAZ, ICAM2, SELL, MAN1A1, SERPINA5, PACSIN2, NCF1B, TMEM59, YWHAB, ABAT, ADH1B, ASL, ASS1, CDH2, CAB39, CPS1, CD226, COL6A3, CUL4A, DSC1, ENTPD5, EIF4A1, FN1, PGC, RHEB, GNAI2, GNB1, GNA13, ITGA2B, ITGB1, ILK, F11R, LTA4H, LIMS1, NAV2, FAM129B, NNMT, NID1, PPIA, PLA1A, PPBP, PECAM1, GP1BB, PCSK9, MENT, SERPINA10, F2RL3, LOX, SFTPB, RAB5B, RALB, REEP6, RETN, AGXT, CCT2, THBD, ISG15, and ZYX.

12. The marker according to claim 11, which is at least one type of protein selected from COPB2, KRAS, PRKCB, RHOC, CD147, CAPN2, ECM1, FGG, and MFAP4.

13. A composition for a prediction of severe COVID-19, comprising one or more types of substance capable of binding to at least one type of protein selected from COPB2, KRAS, PRKCB, RHOC, CD147, CAPN2, ECM1, FGG, MFAP4, ADI1, AK1, MGAT1, CLDN3, CRP, UQCRC2, FGA, FGB, FGL1, GPX1, GSK3B, LBP, PDGFC, RAB13, RAP1B, SLC6A4, UBA7, ORM1, RNPEP, ANGPT1, APOB, B4GALT1, BHMT, CPN1, GNAZ, ICAM2, SELL, MAN1A1, SERPINA5, PACSIN2, NCF1B, TMEM59, YWHAB, ABAT, ADH1B, ASL, ASS1, CDH2, CAB39, CPS1, CD226, COL6A3, CUL4A, DSC1, ENTPD5, EIF4A1, FN1, PGC, RHEB, GNAI2, GNB1, GNA13, ITGA2B, ITGB1, ILK, F11R, LTA4H, LIMS1, NAV2, FAM129B, NNMT, NID1, PPIA, PLA1A, PPBP, PECAM1, GP1BB, PCSK9, MENT, SERPINA10, F2RL3, LOX, SFTPB, RAB5B, RALB, REEP6, RETN, AGXT, CCT2, THBD, ISG15, and ZYX.

14. The composition according to claim 13, comprising one or more types of substance capable of binding to at least one type of protein selected from COPB2, KRAS, PRKCB, RHOC, CD147, CAPN2, ECM1, FGG, and MFAP4.

15. The composition according to claim 14, comprising two or more types of substances capable of binding to the protein(s).

16. The composition according to claim 15, comprising the following combination (a) or (b):
(a) a combination of two or more types selected from a substance capable of binding to PRKCB, a substance capable of binding to RHOC, a substance capable of binding to COPB2, and a substance capable of binding to KRAS,
(b) a combination of two or more types selected from a substance capable of binding to ECM1, a substance capable of binding to CAPN2, a substance capable of binding to FGG, and a substance capable of binding to CD147.

17. The composition according to any one of claims 13 to 16, wherein the substance is an antibody or an antigen binding fragment thereof.

18. A kit for a prediction of severe COVID-19 or a device for a prediction of severe COVID-19, comprising a composition according to any one of claims 13 to 17.

19. A method for measuring a level of one or more types of protein selected from COPB2, KRAS, PRKCB, RHOC, CD147, CAPN2, ECM1, FGG, MFAP4, ADI1, AK1, MGAT1, CLDN3, CRP, UQCRC2, FGA, FGB, FGL1, GPX1, GSK3B, LBP, PDGFC, RAB13, RAP1B, SLC6A4, UBA7, ORM1, RNPEP, ANGPT1, APOB, B4GALT1, BHMT, CPN1, GNAZ, ICAM2, SELL, MAN1A1, SERPINA5, PACSIN2, NCF1B, TMEM59, YWHAB, ABAT, ADH1B, ASL, ASS1, CDH2, CAB39, CPS1, CD226, COL6A3, CUL4A, DSC1, ENTPD5, EIF4A1, FN1, PGC, RHEB, GNAI2, GNB1, GNA13, ITGA2B, ITGB1, ILK, F11R, LTA4H, LIMS1, NAV2, FAM129B, NNMT, NID1, PPIA, PLA1A, PPBP, PECAM1, GP1BB, PCSK9, MENT, SERPINA10, F2RL3, LOX, SFTPB, RAB5B, RALB, REEP6, RETN, AGXT, CCT2, THBD, ISG15, and ZYX in exosomes derived from blood derived from a patient, comprising
contacting a blood sample derived from the patient with the composition according to any one of claims 13 to 18.
